(19) Europäisches Patentamt
European Patent Office
Office européen des brevets

(11) **EP 4 289 927 A1**

(12) **EUROPEAN PATENT APPLICATION**

(43) Date of publication:
**13.12.2023 Bulletin 2023/50**

(21) Application number: **22178451.5**

(22) Date of filing: **10.06.2022**

(51) International Patent Classification (IPC):
**C12M 1/34** *(2006.01)* **C12M 1/36** *(2006.01)*
**G16B 40/00** *(2019.01)*

(52) Cooperative Patent Classification (CPC):
**C12M 41/48; C12M 41/36; C12M 41/38;
C12M 41/44**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Sartorius Stedim Data Analytics AB
903 33 Umeå (SE)**

(72) Inventors:
• **MUELLER, Matthias
37079 Goettingen (DE)**

• **REYMANN, Mathias
34302 Guxhagen (DE)**
• **SCHULZE, Markus
37079 Goettingen (DE)**
• **CORBETT, Brandon
Ontario, L6M 2V9 (CA)**
• **MCCREADY, Christopher
Ontario, L6M 2V9 (CA)**

(74) Representative: **Mewburn Ellis LLP
Aurora Building
Counterslip
Bristol BS1 6BX (GB)**

(54) **CONTROL OF PERFUSION FLOW BIOPROCESSES**

(57) Methods for controlling a perfusion bioprocess are described, the method comprising: controlling the value of one or more controlled variables selected from the number or density of viable cells in the bioreactor, the volume of culture in the bioreactor, and the concentration of one or more metabolites in the bioreactor using one or more control loops each configured to control a non-overlapping subset of the controlled variables by setting the value of one or more manipulated variables selected from the feed flow rate, the bleed flow rate and the permeate flow rate, wherein at least one of the control loops uses a controller that identifies a value of the one or more manipulated variables that optimises a control objective using a prediction of the value of the subset of controlled variables from a dynamic model of the bioprocess describing the rate of change of the one or more controlled variables. Systems, computer readable media implementing such methods, and methods for providing tools to implement such methods, are also provided.

**EP 4 289 927 A1**

Fig. 2

**Description**

Field of the Present Disclosure

The present disclosure relates to computer implemented methods, computer programs and systems for the controlling of perfusion flow bioprocesses. Particular methods, programs and systems of the disclosure use state space models within a controller to determine adjustments of manipulated variables that maintain process stability.

Background

Bioprocesses use biological systems to produce a specific biomaterial, for example a biomolecule with therapeutic effects. This process typically involves placing cells and/or microbes into a bioreactor with media containing nutrients under controlled atmospheric conditions. The media is consumed by the cells and used for growth and other metabolic functions, including production of the specific biomaterial and by-products.

Perfusion operating modes, where fresh media is continuously added to the bioreactor while spent media is continuously removed to maintain a constant working volume while retaining cells in the bioreactor, are becoming increasingly popular both for upstream processes and in the production phase. Indeed, such operating modes can enable to reach high viable cell concentration maintained over prolonged periods of time. However, stable and reliable process performance in terms of cell growth and productivity of the bioprocess is crucially dependent on the precise and robust control of the process throughout its duration, including at least ensuring adequate supply of nutrients.

A simple approach that is often used to control such processes is to control the feed flow rate to maintain a predefined ratio of feed flow rate and viable cell density (VCD, also referred to as "viable cell concentration", VCC). This is commonly referred to as "cell specific perfusion rate" (CSPR) control. Such a control strategy can be implemented as an off-line approach, where the feed flow rate is updated based on off-line VCD measurements or based on predicted VCDs using current growth rates derived from off-line measurements. Such a control strategy can also be implemented as an on-line approach, where an on-line biomass sensor allows to permanently measure the VCD during the process. This value can then be used to adapt the feed flow rate accordingly, thus ensuring true on-line cell-specific perfusion rate (CSPR) control. These approaches have a major drawback that CSPR control requires empirical knowledge of the relation of the used cell culture media with the cultivated organism/cell line, i.e. the identification of a critical CSPR. Thus, their implementation is timeconsuming, labour intensive and generally not directly transferable to other production platforms, e.g., when running perfusion cultivations with different cell lines, organisms and media. Additionally, this approach is unable to account for potential physiological and metabolic changes in long-term cultivations, such as e.g. changes due to genetic drift, as the feed to biomass ratio is kept constant throughout the process.

Therefore, a need exists for a system and method for improved control of perfusion flow bioreactors, which do not suffer from the all of the drawbacks of the prior art.

Summary

According to a first aspect of the disclosure, there is provided a method for controlling a bioprocess comprising a cell culture in a bioreactor, the method including the steps of: controlling the value of one or more controlled variables selected from the number or density of viable cells in the bioreactor, the volume of culture in the bioreactor, and the concentration of one or more metabolites in the bioreactor using one or more control loops each configured to control a non-overlapping subset of the controlled variables by setting the value of one or more manipulated variables selected from the feed flow rate, the bleed flow rate and the permeate flow rate, wherein at least one of the control loops uses a controller that identifies a value of the one or more manipulated variables that optimises a control objective using a prediction of the value of the subset of controlled variables from a dynamic model of the bioprocess describing the rate of change of the one or more controlled variables. The method according to the present aspect comprises receiving a measurement of the value one or more controlled variables or a measurement from which the value of the one or more controlled variables can be derived, and automatically updating the value of one or more terms of the dynamic model of the bioprocess before predicting a state trajectory of the bioprocess, using the measurement or an estimate of the value derived from the measurement.

The method of the first aspect may have any one or any combination of the following optional features.

The dynamic model of the bioprocess may be referred to as "state space model". Such a controller may be referred to as a "model predictive controller" (MPC). The one or more metabolites may be substrates. The concentrations (of metabolites or cells) refer to the concentrations in the bulk fluid unless indicated otherwise. The method may be repeated every time a new measurement is received. The estimate of the value(s) may be obtained using a state observer. The method may comprise updating the value of one or more terms of the dynamic model of the bioprocess selected from a cell growth rate and a substrate uptake rate. The state observer may also comprise a dynamic model of the bioprocess describing the rate of change of the one or more controlled variables. The dynamic model of the bioprocess used by the state observer may be the same model used by the controller.

The dynamic model may comprise one or more equations that describe the change of number and or density of viable cells (kinetic growth model) and/or the change in concentration of ore or more metabolites (e.g. substrates) in the bioreactor and/or the change of the volume of the culture in the bioreactor. The value of one or more terms of the dynamic model of the bioprocess updated may comprise an effective growth rate of cells (also referred to as "growth rate" or "cell growth rate" - this may be strictly speaking an effective growth rate such as e.g. in equation (11) or a growth rate that combines growth and death such as e.g. in equation (26)) and/or a substrate uptake rate (this may be a cell specific substrate uptake rate such as e.g. in equations (25), (25a), (25b) or a coefficient that combines viable cell density and cell specific substrate uptake rate such as in equations (9) or (9a)). The coefficients may be updated by estimating their value as a state variable of the state space model (e.g. by integrating the state space model at pseudo steady state). Thus, the method may comprise estimating the value of one or more terms of the state space model by a state observer and using said estimated value of the one or more coefficients to predict the trajectory of the bioprocess using said dynamic model by said controller.

In embodiments, a state observer may use a model that describes the change in substrate concentration, and may receive measurements for the substrate concentration. The state observer may use these measurements and the model to provide estimates of the current values of one or more state variables (including the substrate concentration) and one or more coefficients of the model such as a substrate uptake rate, which are used by the controller to predict the value of the VCD and substrate concentration using the dynamic model of the bioprocess and one or more candidate values for the manipulated variables (e.g. value of the feed flow), where the manipulated variables may represent inputs of the state space model. The predictions may be evaluated using a control objective in order to identify an optimal control strategy.

In embodiments, a state observer may use a model that describes the change in cell density and substrate concentration, and may receive measurements for viable cell density, and substrate concentration. The state observer may use these measurements and the model to provide estimates of the current values of one or more state variables (including the VCD and substrate concentration) and one or more coefficients of the model such as a growth rate and/or substrate uptake rate, which are used by the controller to predict the value of the VCD and substrate concentration using the dynamic model of the bioprocess and one or more candidate values for the manipulated variables (e.g. values of the feed, bleed and permeate flows), where the manipulated variables may represent inputs of the state space model. The predictions may be evaluated using a control objective in order to identify an optimal control strategy.

In embodiments, a state observer may use a model that describes the change in cell density and volume, and may receive measurements for viable cell density and volume. The state observer may use these measurements and the model to provide estimates of the current values of one or more state variables (including the VCD and volume) and one or more coefficients of the model such as a growth rate, which are used by the controller to predict the value of the VCD and volume using the dynamic model of the bioprocess and one or more candidate values for the manipulated variables (e.g. values of the feed, bleed and permeate flows), where the manipulated variables may represent inputs of the state space model. The predictions may be evaluated using a control objective in order to identify an optimal control strategy.

In embodiments, a state observer may use a model that describes the change in cell density, substrate concentration and volume, and may receive measurements for viable cell density, substrate concentration and volume. The state observer may use these measurements and the model to provide estimates of the current values of one or more state variables (including the VCD, substrate concentration and volume) and one or more coefficients of the model such as a growth rate and substrate uptake rate, which are used by the controller to predict the value of the VCD, substrate concentration and volume using the dynamic model of the bioprocess and one or more candidate values for the manipulated variables (e.g. values of the feed, bleed and permeate flows), where the manipulated variables may represent inputs of the state space model. The predictions may be evaluated using a control objective in order to identify an optimal control strategy.

The model used in the state observer and/or MPC may be automatically updated using measurements from the bioprocess.

Automatically updating the value of one or more terms of the dynamic model may comprise comparing the measurement or an estimate of a state variable of the model derived from said measurement with a corresponding prediction of the dynamic model, and adjusting the value of one or more parameters of the model to reduce (or minimise) the difference between the measurement or estimate of the state variable of the model derived from said measurement and the corresponding prediction of the dynamic model. The use of a dynamic model that is automatically updated is particularly advantageous in the context of control of perfusion bioprocesses because this improves the stability of the controlled bioprocess by more accurately reflecting the evolving behaviour of the bioprocess. This is particularly relevant in perfusion bioprocesses because these can be conducted for prolonged periods of time, over which any of the physiological characteristics of the cells and/or physical parameters of the bioprocess may vary. Automated update of the model enables the model to remain simple while accounting for such changes, rather than needing to use a more complex model that explicitly models the phenomena associated with such changes. As a concrete example, a bioprocess that involves infection of cells with a virus may effectively operate with a varying growth rate as the infection process impacts the growth rate. Enabling a kinetic growth model (used as a dynamic model of the bioprocess) to auto-update to change the effective growth rate during operation of the process is a simple way to account for this, thereby ensuring that the model accurately predicts the internal states of the system throughout the bioprocess, and hence is able to determine appropriate control actions. Failure to do so may result in undesirable cycles in the control actions as the controller may overestimate control actions to be taken. This is simpler than using a kinetic growth model that explicitly accounts for the impact of viral infection on cell growth dynamics. The advantage in terms of simplicity directly translates to improved robustness, improved ease of use as fewer parameters need to be calibrated, and improved transferability of the control method (as the model is applicable to a wide variety of situations).

Controlling the value of the one or more controlled variables may comprise controlling the value of each of: the number or density of viable cells in the bioreactor, the volume of culture in the bioreactor, and the concentration of one or more metabolites in the bioreactor. Controlling the value of the one or more controlled variables may comprise setting the value of each of: the feed flow rate, the bleed flow rate and the permeate flow rate. The one or more metabolites may be a substrate, such as e.g. glucose. This may be used as a control objective alone or in combination with a media exchange rate. Thus, the control objective may comprise a term that penalises deviations from targets for one or more substrates and/or targets for a media exchange rate. This may improve the stability of the bioprocess by ensuring that cells are provided with adequate amounts of nutrients throughout the operation of the bioprocess while reducing potential waste of nutrient through excessive media exchange rates.

The control objective may be an expression that penalises: differences between the prediction of the value of the subset of controlled variables and corresponding predetermined target values, and changes in the value of the one or more manipulated variables. The control objective may be an expression that comprises any one or more of the terms of equations (1), (6), (7) or (8) (or equations derived therefrom such as e.g. equations (1a)-(1f), (6a)-(6e), (7a)-(7e), (8a)-(8e)). For example, when the control loop controls the concentration of a metabolite by manipulating the feed flow, the control objective may only comprise the terms related to the concentration of metabolite and feed flow in equation (1). The use of a control objective that penalises changes in the value of the one or more manipulated variables improves the stability or the controlled process compared to methods that use conventional controllers. In other words, the model predictive controller has as one of its objectives to maximise stability (minimise changes in the values of manipulated variables). This is by contrast to conventional approaches to controlling perfusion flow bioprocesses where the process is controlled to keep the VCD and/or the media exchange ratio as close as possible to predetermined targets by making aggressive adjustments so those targets are met.

The control objective may be configured to penalise changes in the value of the one or more manipulated variables more strongly than differences between the prediction of the value of the subset of controlled variables and corresponding predetermined target values. The control objective may be configured to prioritise stability of the manipulated variables over proximity to predetermined target values. The control objective may weigh at least equally (or about equally) a term that penalises changes in the value of the one or more manipulated variables and a term that penalises differences between the prediction of the value of the subset of controlled variables and corresponding predetermined target values. In other words, the control objective may be configured such that the controller selects smooth control actions /makes slow gradual adjustments to make the bioprocess gradually drift to the one or more target values for the controlled variables. The control objective may thus be calibrated to give the controller freedom to deviate from setpoints in order to make slow adjustments that gradually drift to setpoints. This is by contrast to conventional models where some variables are aggressively controlled, such as e.g. the volume of liquid in the bioreactor. Smooth / gradual input actions may be beneficial for the cell culture are cells generally perform better in stable environments (in terms of e.g. growth, productivity, etc.).

The control objective may comprise at least one term that penalises predicted values of a controlled variable that are below a predetermined first threshold. The term that penalises predicted values of a controlled variable that are below a predetermined first threshold may penalise the difference between the predicted values of the controlled variable and the predetermined first threshold if said predicted values are below said predetermined first threshold. The control objective may comprise at least one term that penalises predicted values of a controlled variable that are above a predetermined second threshold. The term that penalises predicted values of a controlled variable that are above a predetermined second threshold may penalise the difference between the predicted values of the controlled variable and the predetermined second threshold if said predicted values are above said predetermined second threshold. Thus, the objective function may be configured to include constraints that apply to thresholds (i.e. values that are best not exceeded / undershot by the controlled variables), for example where stronger control actions may be taken, as well as constraints that apply to targets (i.e. values that are ideally approached by the controlled variables). The control objective may weight the terms that penalise predicted values of a controlled variables that are below/above respective thresholds more than terms that penalise differences between predicted values of a controlled variable and corresponding predetermined targets. This may result in hard and soft constraints, where larger control actions may be selected by the controller to avoid penalties of the first kind than to avoid penalties of the second kind. This may be useful for example where physical constraints are such that some values of a controlled variable would be detrimental to the bioprocess. For example, when using volume as a controlled variable it may be advantageous to allow for subtle adjustments to the volume around a target, while selecting more drastic control actions to control the volume if this is predicted to exceed a maximum volume (e.g. to avoid overflow). The control objective may be an expression that comprises any one or more of the terms of equation (7). For example, when the control loop controls the concentration of a metabolite by manipulating the feed flow, the control objective may only comprise the terms related to the concentration of metabolite and feed flow in equation (7).

The one or more controlled variables may comprise the viable cell density, and the control objective may use the corresponding total number of cells instead of the viable cell density as control variable. The control objective may comprise a term that penalises differences between the prediction of the value of the total number of cells in the bioprocess and corresponding predetermined target values, instead or in addition to a term that penalises differences between the prediction of the value of the viable cell density in the bioprocess and corresponding predetermined target values. The control objective may be an expression that comprises any one or more of the terms of equation (6). The control objective may comprise a term that penalises predicted values of the total cell number that are below a predetermined first threshold, and/or a term that penalises predicted values of the total cell number that are above a predetermined second threshold. The first and/or second threshold and/or the predetermined target values may be determined from thresholds/target values for the viable cell density using measured, set, estimated or predicted values for the volume of the culture at the time of the prediction.

The method may comprise using a state observer to provide an estimate of the value of one or more state variables of the bioprocess, the one or more state variables of the bioprocess comprising the one or more controlled variables, using one or more measurements of the state variables of the bioprocess and a dynamic model of the bioprocess describing the rate of change of said state variables. The state observer may provide an estimate of the value of one or more state variables of the bioprocess using a plurality of measurements for at least one of the state variables of the process, wherein the plurality of measurements include a first measurement and a second measurement, wherein the first measurement is acquired more frequently than the second measurement. The state observer may provide an estimate of the value of one or more state variables of the bioprocess using a measurement that is acquired with a time delay and wherein the state observer provides an estimate of the value of one or more state variables of the bioprocess that takes into account said time delay. The one or more state variables may comprise at least one variable selected from: a consumption rate of a substrate, a production rate of product, a specific transport rate of a metabolite (where the metabolite can be a substrate or a product), a bulk fluid concentration of a metabolite (where the metabolite can be a substrate or a product), the bulk fluid concentration of a biomaterial that accumulates in the culture as a result of cell growth and inhibits cell growth, a viable cell density, a dead cell density, a total cell density, a cell viability, an effective growth rate of cells, a death rate of cells and lysed cell density. The one or more state variables may comprise any variable of a dynamic model of the bioprocess describing the evolution of one or more of the controlled variables. For example, the one or more state variables may comprise any one or more of the variables of equations (9)-(10) and (25) and equivalents thereof. The dynamic model may comprise one of more further state variables in addition to the one of more state variables that are controlled variables. For example, a dynamic model comprising an equation describing the rate of change of the concentration of a substrate in the bulk fluid may have a state variable that is the concentration of the substrate in the bulk fluid and a state variable that is the rate of consumption of the substrate. The rate of consumption of the substrate may represent consumption by the cells in the bioprocess as well as any loss through harvest and/or bleed flows. The dynamic model used in the controller and in the state observer may be the same model. The state observer may use a plurality of measurements for at least one of the state variables of the process, wherein the plurality of measurements include a first measurement and a second measurement, wherein the first measurement is acquired more frequently than the second measurement. For example, the state observer may use the most recent value of the first measurement and the second measurement, wherein the most recent value of the second measurement may be the same value for a plurality of iterations and the most recent value of the first measurement may be different at every iteration of the control method. For example, the first measurement may be obtained using an on-line sensor and the second measurement may be obtained using an off-line sensor.

The method may comprise identifying one or more values of the one or more manipulated variables that minimises the control objective over a time period, and controlling the bioprocess to use one of the one or more values for each of the one or more manipulated variables. The controller that identifies a value of the one or more manipulated variables that optimises a control objective may control two or more controlled variable using a prediction of the value of the subset of controlled variables from a dynamic model of the bioprocess jointly describing the rate of change of the two or more controlled variables, and measurements of the value of the two or more controlled variables or estimates of the value of said variables obtained from said measurements. The use of a MPC that jointly predicts two controlled variables and regulates the process based on measurements of both of these variables may be advantageous over independent control of said controlled variables as such a control method is more robust to failure of the measurement systems used to measure either one of the two variables. This may be particularly advantageous when one of the measurements is difficult to obtain or obtainable using sensor systems that are prone to failure. For example, measurements for substrates / metabolites may be more difficult to obtain than measurements for viable cell density. As such, coupling regulation of a substrate / metabolite with regulation of VCD as described herein may improve the robustness of the substrate / metabolite regulation process.

Identifying a value of the one or more manipulated variables that optimises a control objective may comprise using the dynamic model of the bioprocess to predict a state trajectory of the bioprocess with one or more candidate values of the one or more manipulated variables. This may be performed by finding values of the one or more state space variables that represent a solution of the dynamic model at one or more future time points. Identifying a value of the one or more manipulated variables that optimises a control objective may comprise using the prediction of the value of the subset of controlled variables in said state trajectory as variables of the control objective. The method may comprise selecting and comparing candidate values using an optimisation algorithm. The state observer may be a Kalman filter, a Luenberg observer or a moving horizon estimation. The controlled variables may comprise the viable cell density, volume and concentration of one or more metabolites. The manipulated variables may comprise the feed flow rate, the permeate flow rate, and the bleed flow rate. The one or more control loops may comprise: a control loop that uses a controller that identifies a value of the one or more manipulated variables that optimises a control objective using a prediction of the value of the subset of controlled variables from a dynamic model of the bioprocess describing the rate of change of the one or more controlled variables; and one or more further control loops, optionally wherein the one or more further control loops do not use a dynamic model of the bioprocess and/or wherein the one or more further control loops use a controller selected from a proportional controller, a proportional-integral controller, and a proportional-integral-derivative controller.

The manipulated variables may include the permeate flow rate and the control objective comprises a term that penalises differences between candidate permeate flow rates and corresponding predetermined target values, a term that penalises candidate values of the permeate flow rate that are below a predetermined first threshold, and/or a term that penalises candidate values of the permeate flow rate that are above a predetermined second threshold. The control objective may comprise a term that penalises differences between candidate media exchange rates and corresponding predetermined target values, a term that penalises candidate values of the media exchange rate that are below a predetermined first threshold, and/or a term that penalises candidate values of the media exchange rate that are above a predetermined second threshold. The control objective may be an expression that comprises any one or more of the terms of equation (8).

The subset of controlled variables controlled by the control loop that uses a controller that identifies a value of the one or more manipulated variables that optimises a control objective using a prediction of the value of the subset of controlled variables from a dynamic model of the bioprocess describing the rate of change of the one or more controlled variables may comprise: (i) the concentration of one or more substrates, and/or (ii) the volume and viable cell density, optionally wherein the one or more manipulated variables comprises the permeate flow rate, the bleed flow rate and the feed flow rate. When the subset of controlled variables comprises the concentration of one or more substrates, the one or more manipulated variables may comprise the feed flow rate. The one or more control loops may further comprise a control loop, optionally using a P, PI or PID controller, that controls the volume as controlled variable using the permeate flow rate as manipulated variable, and a control loop, optionally using a P, PI or PID controller, that controls the viable cell density as controlled variable using the bleed flow rate as manipulated variable.

The method may further comprise obtaining a measurement for one or more of the controlled variables or a variable from which one or more of the controlled variables can be derived. The method may further comprise repeating the control method at one or more time points during operation of the bioprocess to determine corresponding values for the one or more manipulated variables. The method may further comprise repeating the control method at regular intervals during operation of the bioprocess to determine corresponding values for the one or more manipulated variables. The method may further comprise receiving a set of measurements for one or more of the controlled variables or variables from which one or more of the controlled variables can be derived and determining, using the one or more controller loops and the measurements, values of the manipulated variables, optionally wherein the step of determining is repeated every time a new set of measurements is received. The method may further comprise providing a set of values of the manipulated variables determined by the one or more control loops to a user, user interface, computer or actuator. The method may further comprise providing a signal to a control unit for control of one or more effectors to set the values of one or more manipulated variables according to a value determined by the one or more control loops. The method may further comprise receiving the value of one or more parameters of the controller, optionally from a computer device, data store, or user through a user interface, optionally wherein the parameters include parameters selected from: one or more predetermined targets for one or more controlled variables (such as e.g. set points for the one or more controlled variables; one or more of the predetermined targets may be variable during the course of the bioprocess, i.e. a different set point may be provided for each of a plurality of time points of the bioprocess), one or more parameters of the bioprocess (such as e.g. parameters related to the physical configuration of the bioprocess, physical constraints of the bioprocess, characteristics of the cell culture, temperature, etc.), one or more parameters of a dynamic model of the bioprocess (such as e.g. coefficients, initial values, etc.), one or more predetermined thresholds for one or more controlled variables (such as e.g. low/high thresholds below/above which a penalty is included in the objective function), a selection of one or more controlled variables, a selection of one or more manipulated variables, the value of one or more parameters of the control objective (such as e.g. the value of the weights applied to any of the terms of the control objective), and a selection of a state observer model (such as e.g. a Kalman Filter, a moving horizon estimator, a particular cost function for the moving horizon estimator, a dynamic model of the bioprocess to be used by the state observer, one or more parameters of the state observer such as the gain of a Kalman Filter, etc.), a time period for integration of the dynamic model of the bioprocess, and a frequency of iteration of the control method. The method may further comprise providing to a computing device, data store or user through a user interface, a prediction of the value of one or more states of the bioprocess determined by integration of the dynamic model of the bioprocess. In other words, the control method may be iterative.

The method may further comprise selecting the value one or more parameters selected from: parameters of the dynamic model used by the controller and/or state observer, parameters of the state observer, and parameters of the control objective, using a simulation of the state trajectory of a bioprocess to model the response of the bioprocess to control actions selected by the controller. Methods for simulating the state trajectory of a bioprocess may be as described in WO 2021/165495. Selecting the value of said parameters may comprise comparing the state trajectories of the bioprocess and the control actions selected by the controlled using a first set of parameters and a second set of parameters, optionally using different sets of initial conditions, and selecting the set of parameters that performs best in terms of stability of the model and compliance to the control objective, optionally under a plurality of initial conditions (i.e. the set of parameters that results in a robust model). The present inventors have found that the control methods described herein are generally extremely robust and that there was no need to recalibrate the models and controllers at every use, especially when the models are automatically updated as described above. In particular, the weights of the control objective (parameters that weight the relative importance of the different terms of the control objective) may be set to ensure stability in one test or simulated bioprocess, and used without modification for any other bioprocess. In particular, all weights of the control objective may be set to 1. Alternatively, the control objective may weigh a term that penalises changes in the value of the one or more manipulated variables at least twice, 5 times, 10 times or 100 times more than a term that penalises differences between the prediction of the value of the subset of controlled variables and corresponding predetermined target values. For example, a term that penalises changes in the value of the one or more manipulated variables may be set a weight of 2, 5, 10 or 100, and a term that penalises differences between the prediction of the value of the subset of controlled variables and corresponding predetermined target values may be set a weight of 1. The control objective may comprise quadratic terms. All terms of the control objective may be quadratic. The dynamic model of the bioprocess may comprise one or more material balance equations. Material balance equations may be useful to describe the rate of change of the volume in the bioreactor and/or the rate of change of the concentration of one or more metabolites. For example, for each of one or more metabolites, a dynamic model may comprise an equation of the form given by equation (25) or equation (9).

The dynamic model may comprise one or more equations of a kinetic growth model. A kinetic growth model may comprise one or more equations representing changes in one or more of the viable cell density $x_v$, the dead cell density $x_d$, the total cell density $x_t$ and the lysed cell density $x_l$ of the cell culture as a function of time. The kinetic growth model may comprise a Monod kinetic model or a saturation kinetic model. The kinetic growth model may be configured to estimate microbial cell growth as a function of time. For example, any or all of equations (11) to (14), or equations derived therefrom, may be used. For example, equations may be derived from these by: substituting one or more terms in equations (11)-(13) by a corresponding term derived from equation (14), removing one or more terms of any of equations (11)-(14) in line with corresponding assumptions (such as e.g. where a particular flow is assumed to be absent), or adding one or more terms of any of equations (11)-(14) in line with corresponding assumptions (such as e.g. where an additional flow is assumed to be present). In equations (11)-(13), the terms $\mu_{eff}$ and/or $\mu_d$ may be modeled using any of equations (15)-(24). A material balance model may comprise one or more equations representing changes in the bulk concentration of one or more metabolites in the bioreactor.

A bulk fluid concentration of a metabolite may be obtained using material balance equations, such as equation (25) or equivalents thereof. A viable cell density may be obtained using equation (11) or equivalents thereof. A cell viability may be obtained as the ratio of the viable and total cell densities, using equations (11)-(14) or equivalents thereof. A dead cell density may be obtained using equation (12) or equivalents thereof. A lysed cell density may be obtained using equation (13) or equivalents thereof. The bulk fluid concentration of a biomaterial that accumulates in the culture as a result of cell growth and inhibits cell growth may be obtained using equation (16) or equivalents thereof. The bulk fluid concentration of a product may be obtained using material balance equations, such as equation (25) or equivalents thereof. An effective growth rate may be obtained using equation (22) or equivalents thereof. A death rate may be obtained using equation (15) or equivalents thereof. Equivalents of the equations provided may refer to equations that capture the same processes but differ by one or more underlying model assumptions.

Obtaining the values of one or more state variables of a dynamic model of the bioprocess (also referred to as "state space model") at one or more time points may comprise predicting a state trajectory of the bioprocess using the state space model. Predicting a state trajectory of the bioprocess using the state space model may comprise finding values of the one or more state space variables that represent a solution of the state space model at the one or more maturities. In other words, obtaining the values of one or more state variables of a state space model or variables derived therefrom at one or more time points may comprise solving the state space model at the or more time points, for example by integrating one or more equations that together form the model, or using any other suitable approach depending on the model such as e.g. stochastic simulation. The state of the process may include the value of any of the variables in such models, such as e.g. the value of one or more cell culture state variables (e.g. live, dead, lysed or total cell densities), the volume of the culture and/or metabolite concentrations. The state trajectory of the process may include the value of state variables at a plurality of time points. Thus, the concentration of a metabolite i (or any other controlled variable that may be measured to provide feedback to the controllers) may be seen as an output of the bioprocess (where inputs are manipulated variables) and as a state variable of the state space model. Predicting a state trajectory of the bioprocess using the state space model may comprise determining the value of the one or more state space variables at a single time point, or at a plurality of time points. In other words, a trajectory may comprise values for a set of state variables at a single time point. According to a second aspect, there is provided a method for controlling a bioprocess comprising a cell culture in a bioreactor, wherein the bioprocess is operated as a perfusion bioprocess, the method comprising: controlling the value of a plurality of controlled variables selected from the number or density of viable cells in the bioreactor, the volume of culture in the bioreactor, and the concentration of one or more metabolites in the bioreactor using one or more control loops each configured to control a non-overlapping subset of the controlled variables by setting the value of one or more manipulated variables selected from the feed flow rate, the bleed flow rate and the permeate flow rate, wherein at least one of the control loops uses a controller (which may be referred to as "predictive controller") that jointly controls a plurality of controlled variables, wherein the controller is configured to identify values of a plurality of manipulated variables that optimise a control objective using a prediction of the value of the subset of controlled variables from a dynamic model of the bioprocess describing the rate of change of the one or more controlled variables. In other words, the subset of controlled variables controlled by said predictive controller may include two or more of: the number or density of viable cells in the bioreactor, the volume of culture in the bioreactor, and the concentration of one or more metabolites in the bioreactor. The method according to the present aspect may have any of the features disclosed in relation to the first aspect. The method of the present aspect may further have any one or any combination of the following optional features.

The plurality of controlled variables controlled by said predictive controller may include: the number or density of viable cells in the bioreactor, and the volume of culture in the bioreactor. The manipulated variables used by said predictive controller may include the feed, bleed and harvest/permeate flows. The plurality of controlled variables controlled by said predictive controller may include: the number or density of viable cells in the bioreactor, and the concentration of at least one metabolite (preferably a substrate, such as e.g. glucose) in the bioreactor. The manipulated variables used by said predictive controller may include the feed, bleed and harvest/permeate flows. The plurality of controlled variables controlled by said predictive controller may include: the number or density of viable cells in the bioreactor, the volume of culture in the bioreactor, and the concentration of at least one metabolite (preferably a substrate, such as e.g. glucose) in the bioreactor. The manipulated variables used by said predictive controller may include the feed, bleed and harvest/permeate flows.

All of the steps of the methods described herein are computer implemented unless context indicates otherwise. In particular, any of the steps of the present method may be implemented by a computing device, optionally in operable communication with one or more sensors, other computing devices and/or user interfaces.

According to a third aspect, there is provided a system for controlling a bioprocess, the system including: at least one processor; and at least one non-transitory computer readable medium containing instructions that, when executed by the at least one processor, cause the at least one processor to perform operations comprising: the steps of any method of any preceding aspect. In particular, the at least one non-transitory computer readable medium may contain instructions that, when executed by the at least one processor, cause the at least one processor to perform operations comprising any of the operations described in relation to the first and second aspect.

According to a further aspect, there is provided a non-transitory computer readable medium comprising instructions that, when executed by at least one processor, cause the at least one processor to perform the method of any embodiment of any aspect described herein.

According to a further aspect, there is provided a computer program comprising code which, when the code is executed on a computer, causes the computer to perform the method of any embodiment of any aspect described herein.

Brief Description of the Drawings

Embodiments of the present disclosure will now be described by way of example with reference to the accompanying drawings in which:

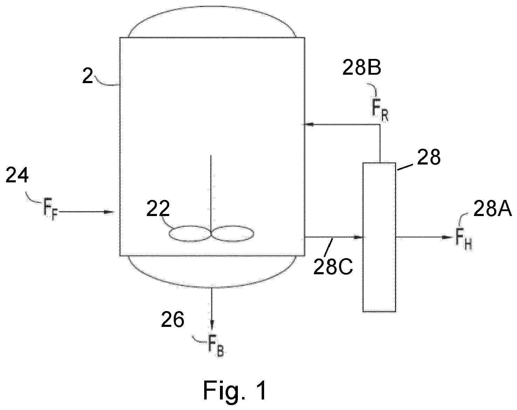

> **Figure 1** shows a simplified process diagram for a generic bioprocess according to embodiments of the present disclosure;

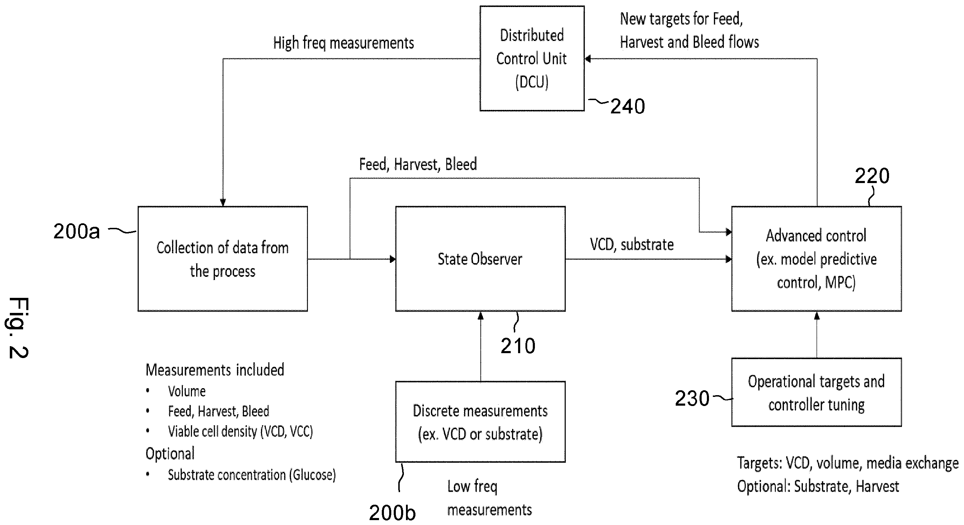

> **Figure 2** illustrates schematically the logic flow according to an embodiment of a control method of the disclosure;

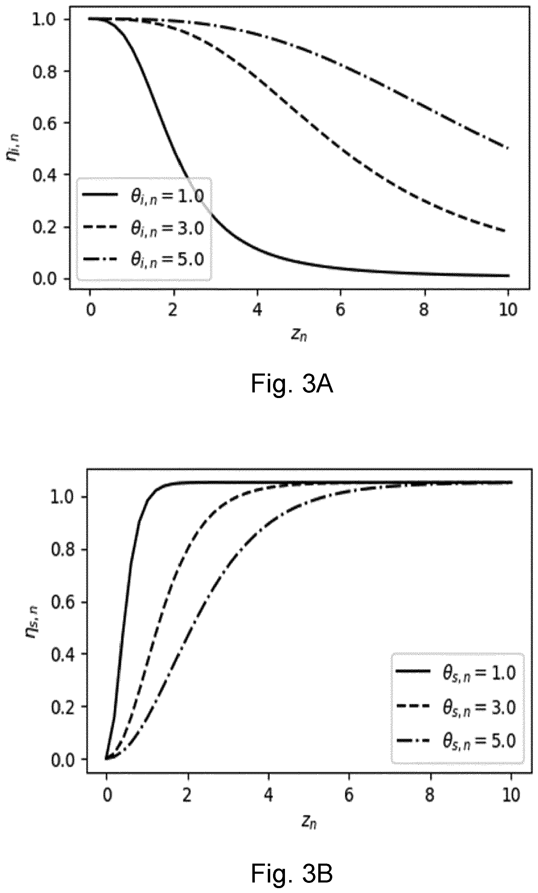

> **Figure 3** illustrates the impact of selected parameters on variables of a kinetic growth model according to embodiments of the present disclosure; Figure 3A is a plot showing the value of a correction factor (y-axis) capturing the effect of a growth inhibition variable on growth rate as a function of the value of the growth inhibiting variable (x-axis, e.g. concentration of a metabolite that has a cytostatic effect) for three exemplary values of the parameter $\theta_{i,n}$ which in this example represents the approximate value of the variable $z_n$ above which the variable starts to inhibit growth; Figure 3B is a plot showing the value of a correction factor (y-axis) capturing the effect of a substrate limiting variable on growth rate as a function of the value of the substrate limiting variable (x-axis, e.g. concentration of a metabolite such as a nutrient) for three exemplary values of the parameter $\theta_{s,n}$ which in this example represents the approximate value of the variable $z_n$ below which the variable starts to have a limiting effect on growth;

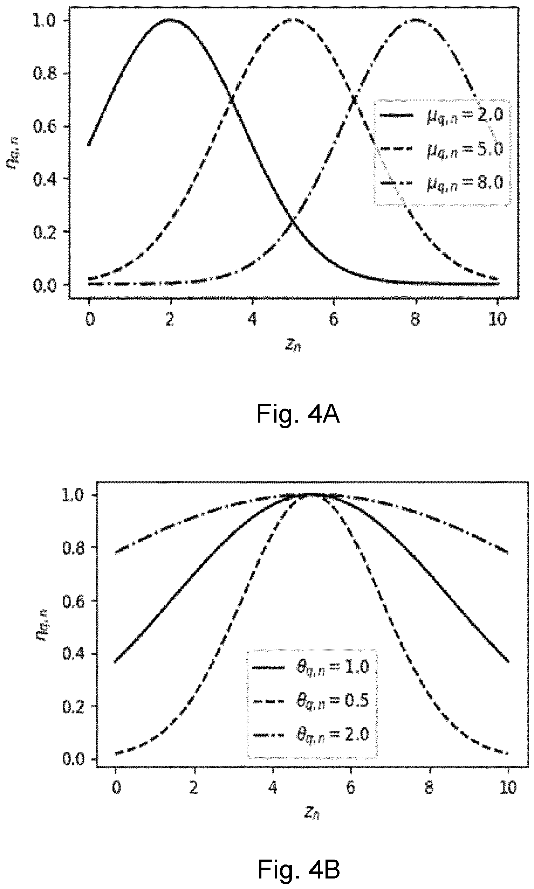

> **Figure 4** illustrates the impact of selected parameters on variables of a kinetic growth model according to embodiments of the present disclosure; Figures 4A and 4B are a plots showing the value of a correction factor (y-axis) capturing the effect of a variable that has a quadratic effect on growth as a function of the value of the of the quadratic effect variable (x-axis, e.g. temperature, pH) for three exemplary values of the parameter parameter $\theta_{q,n}$ at a fixed value of $\mu_{q,n}$ (D) and three exemplary values of the parameter $\mu_{q,n}$ at a fixed value of $\theta_{q,n}$ (C), where in these examples parameter $\theta_{q,n}$ represents the spread of the effect and parameter $\mu_{q,n}$ represents the value at which maximum growth occurs;

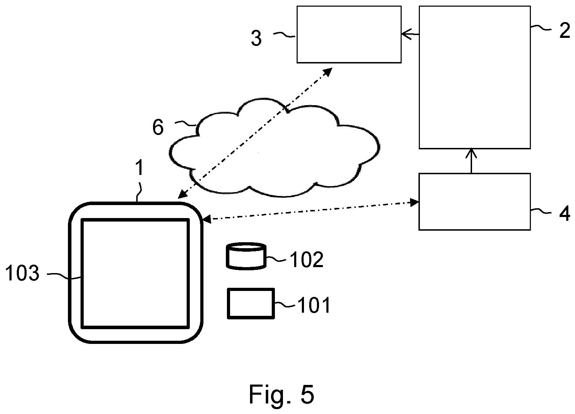

> **Figure 5** illustrates schematically a system according to embodiments of the present disclosure;

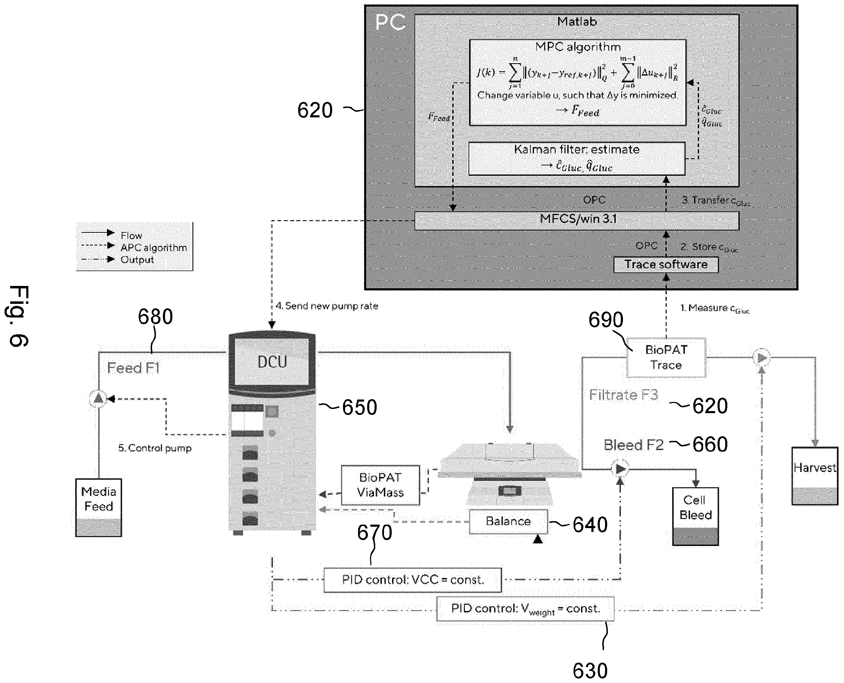

> **Figure 6** illustrates schematically an example of a control system and algorithm (advance predictive control, APC) for control of glucose and viable cell concentration (VCC) in a perfusion flow bioprocess according to the disclosure;

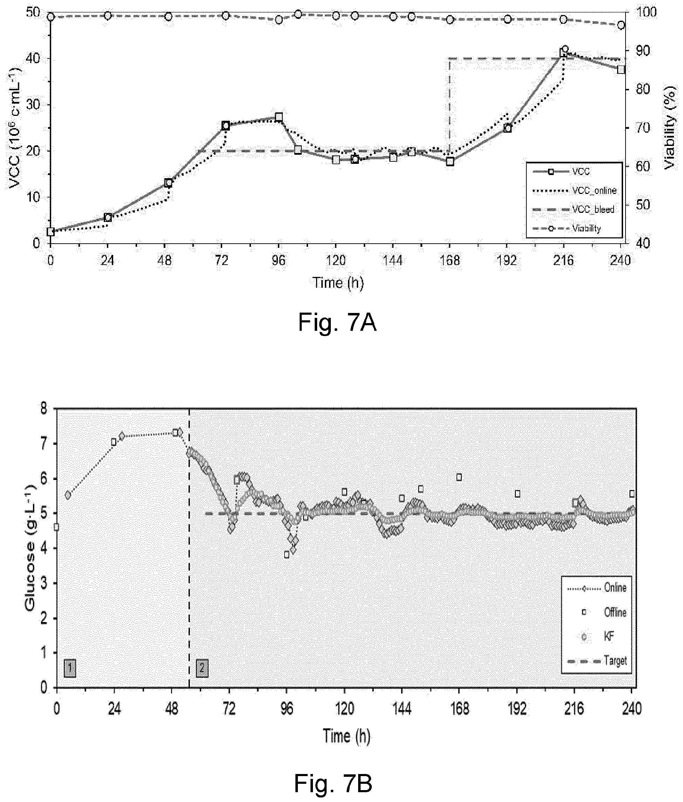

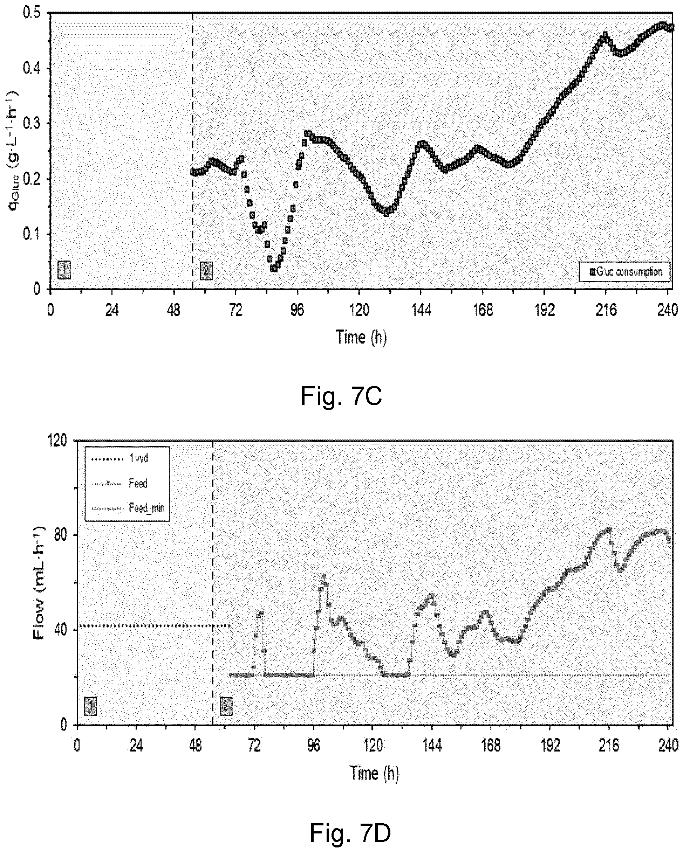

> **Figure 7** shows the results of the use of the system of Figure 6 to control a perfusion flow bioreactor with a constant glucose and two different VCC setpoints. **A.** Off- and on-line VCC, bleed setpoint and viability profile. **B.** Glucose concentrations for off- and on-line values, the Kalman filter (KF) estimates and the glucose target. **C.** Glucose uptake rate derived during the experiment from the APC algorithm. **D.** perfusion rate, i.e. feed flow rate, during the cultivation controlled by the advance predictive control algorithm.

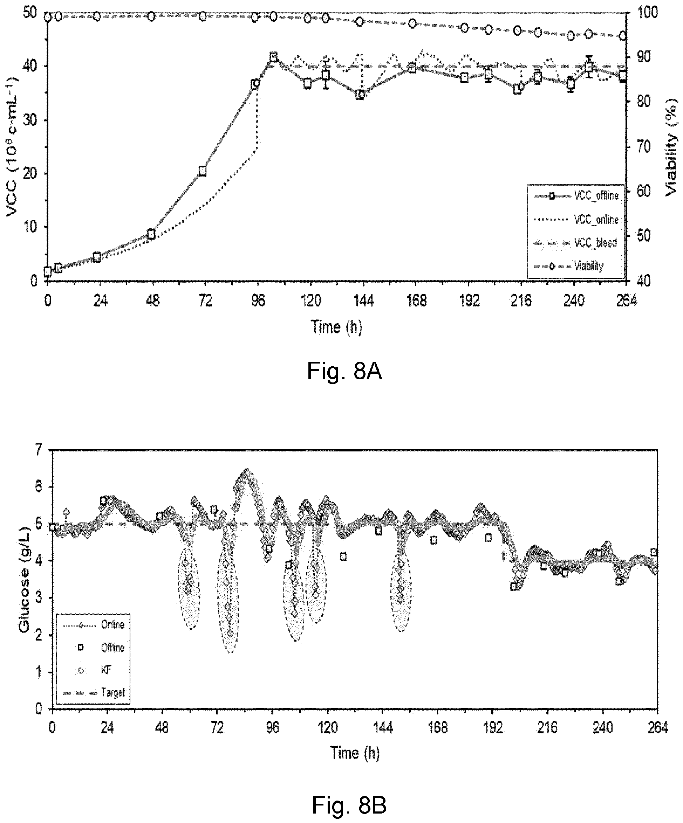

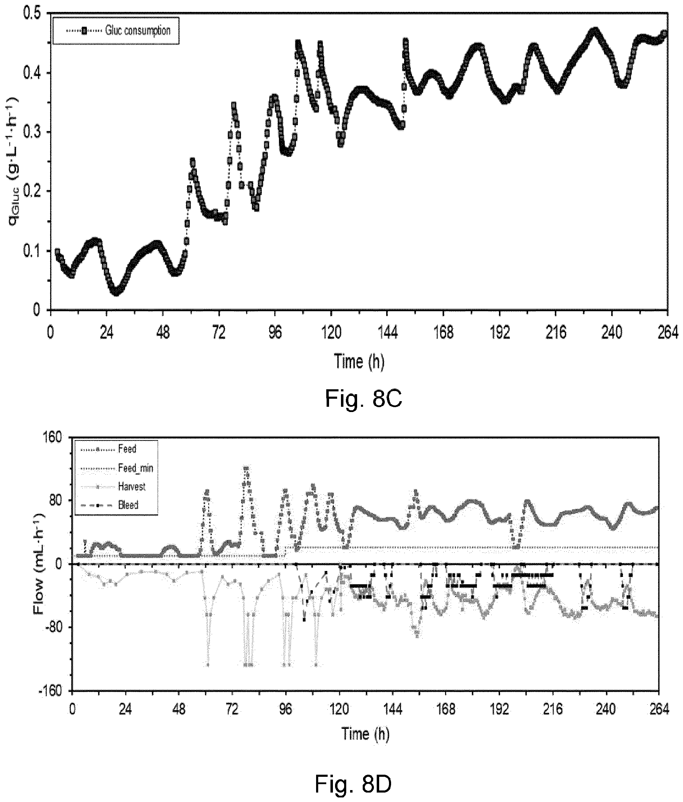

> **Figure 8** shows the results of the use of the system of Figure 6 to control a perfusion flow bioreactor with two different glucose setpoints applied to one VCC setpoint. **A.** Off- and on-line VCC, bleed setpoint and viability profile. **B.** Glucose concentrations for off- and on-line values, the Kalman filter (KF) estimates and the glucose target. C. Glucose uptake rate derived during the experiment from the APC algorithm. **D.** perfusion rate, i.e. feed flow rate, during the cultivation controlled by the APC algorithm.

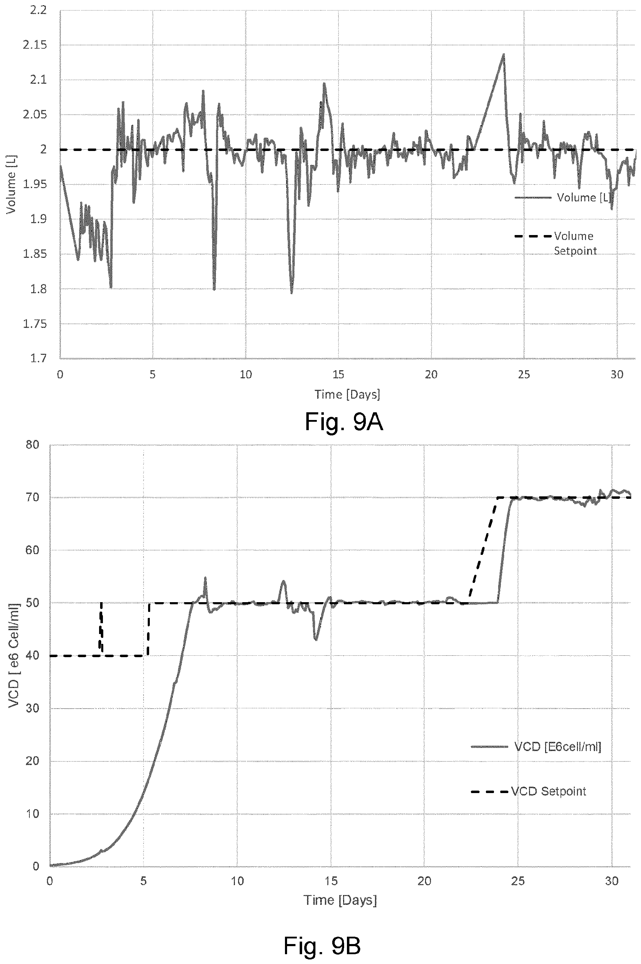

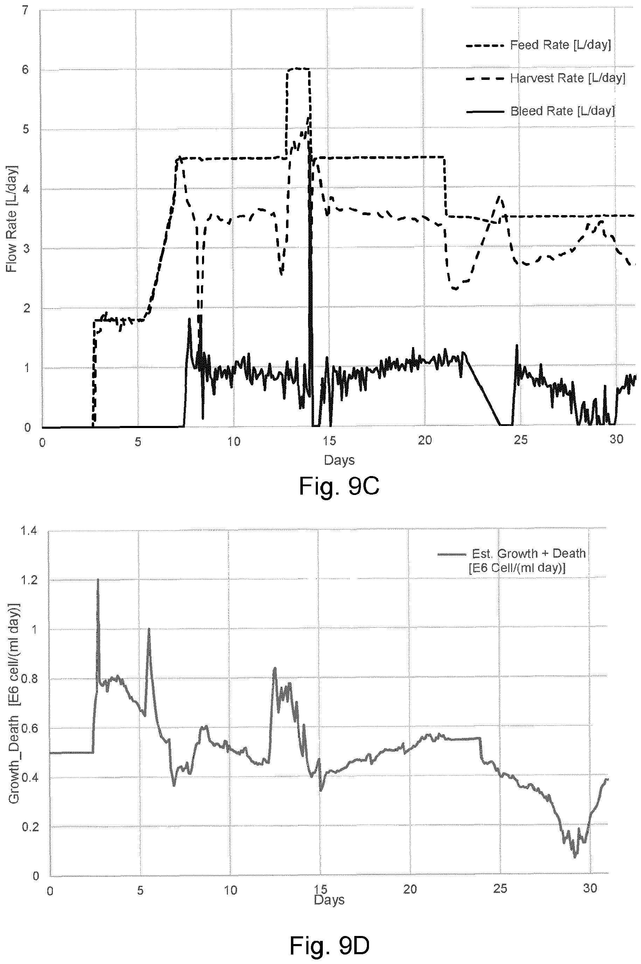

> **Figure 9** shows the results of the use of a control method and system as described herein to control a perfusion flow bioreactor with two different VCD setpoints, using an APC algorithm for joint control of the volume and VCD. **A.** Volume profile and setpoint target. **B.** Viable cell density profile and setpoint targets. **C.** Feed, harvest and bleed flow rates determined by the APC algorithm. **D.** Estimated combined growth/death rate $\mu_{tot}$, calculated using a Luenberg observer.

Where the figures laid out herein illustrate embodiments of the present invention, these should not be construed as limiting to the scope of the invention. Where appropriate, like reference numerals will be used in different figures to relate to the same structural features of the illustrated embodiments.

Detailed Description

Specific embodiments of the invention will be described below with reference to the figures.
As used herein, the terms "computer system" includes the hardware, software and data storage devices for embodying a system or carrying out a method according to the above described embodiments. For example, a computer system may comprise one or more processing units such as a central processing unit (CPU) and/or a graphical processing unit (GPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network. For example, a computer system may be implemented as a cloud computer.

The methods described herein may be provided as computer programs or as computer program products or computer readable media carrying a computer program which is arranged, when run on a computer, to perform the method(s) described herein. The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

**Bioprocess**

As used herein, the term "bioprocess" (also referred to herein as "biomanufacturing process") refers to a process where biological components such as cells, parts thereof such as organelles or multicellular structures such as organoids or spheroids are maintained in a liquid medium in an artificial environment such as a bioreactor. In the context of the present disclosure, a bioprocess typically refers to a cell culture. A bioprocess typically results in a product, which can include biomass and/or one or more compounds that are produced as a result of the activity of the biological components. For example, in upstream processes the aim is typically to obtain biomass. This can then be used in a fermentation stage (cell culture), to produce one or more desired products by the live cells in culture. The one or more desired products may be extracted from the cells or the culture medium in a downstream process. A downstream process may comprise one or more cells, such as separation and/or purification steps. A bioreactor can be a single-use vessel or a multi-use vessel in which a liquid medium suitable for carrying out a bioprocess can be contained. Example bioreactor systems suitable for bioprocesses are described in US 2016/0152936 and WO 2014/020327. For example, a bioreactor may be chosen from: advanced microbioreactors (such as e.g. Ambr® 250 or Ambr® 15 bioreactors from The Automation Partnership Ltd.), single use bioreactors (e.g. bag-based bioreactors such as Biostat® STR bioreactors from Sartorius Stedim Biotech GmbH, available in 50 to 2000: capacity), stainless steel bioreactors (such as e.g. Stainless Steel D-DCU bioreactors available in capacities from 10 to 200L or Cplus available in capacities from 5 to 30L, all from Sartorius Stedim Systems GmbH), benchtop bioreactors (such as e.g. Biostat® B and B-DCU from Sartorius Stedim Systems GmbH, which support either 2L single-use rigid wall vessel or 1, 2, 5 and 10L glass vessels), etc. The present invention is applicable to any type of bioreactor and in particular to any vendor and any scale of bioreactor from benchtop systems to manufacturing scale systems.

A cell culture refers to a bioprocess whereby live cells are maintained in an artificial environment such as a bioreactor. The methods, tools and systems described herein are applicable to bioprocesses that use any types of cells that can be maintained in culture, whether eukaryotic or prokaryotic. The invention can in particular be used to monitor and/or control bioprocesses using cells types including but not limited to mammalian cells (such as Chinese hamster ovary (CHO) cells, human embryonic kidney (HEK) cells, Vero cells, etc.), non-mammalian animal cells (such as e.g. chicken embryo fibroblast (CEF) cells), insect cells (such as e.g. *D. melanogaster* cells, *B. mori* cells, etc.), animal cells of any cell type (such as e.g. induced pluripotent stem cells (iPSCs), stem cells such as e.g. mesenchymal stem cells, immune cells such as T cells and natural killer cells), bacterial cells (such as e.g. *E. coli* cells), fungal (e.g. yeast) cells (such as e.g. S. *cerevisiae* cells), and plant cells (such as e.g. *A. thaliana* cells). A bioprocess typically results in the production of a product, which can be the cells themselves (e.g. a cell population for use in further bioprocesses, a cell population for use in cell therapy, a cell population for use as a product such as a probiotic, feedstock, etc.), a macromolecule or macromolecular structure such as a protein, peptide, nucleic acid or viral particle (e.g. a monoclonal antibody, immunogenic protein or peptide, a viral or non-viral vector for gene therapy, enzymes such as e.g. for use in the food industry, for environmental applications such as water purification, decontamination, etc.), or a small molecule (e.g. alcohols, sugars, amino acids, etc.).

**Figure 1** shows a simplified process diagram for a generic bioprocess. The bioprocess is implemented in the reactor 2, which in the embodiment shown is equipped with agitation means 22. Four flows (also referred to herein as "streams") are depicted, although depending on the particular situation any or all of these flows may be absent. A first flow 24 is a feed flow $F_F$ (also referred to herein as "perfusion flow") containing anything that is added to the culture in the bioreactor (typically this includes fresh medium, in which case the bioprocess may be referred to as a "fed-batch" process, "perfusion" process or "continuous" process), a second flow 26 is a bleed flow $F_B$ that has the same composition as the culture in the bioreactor, a third flow 28A is a permeate $F_H$ (also referred to as "harvest flow" or "harvest stream") which is obtained through the processing of an auxiliary harvest stream 28C through a cell retention device 28 (CRD, which can be any cell separation means adapted to separate cells from medium) to produce the third (harvest) and fourth flow 28B, the fourth flow 28B being a recycle flow $F_R$ (also referred to as "retentate") comprising the cells and any culture medium that has not been completely separated out in the cell retention device 28. In embodiments, the recycle flow $F_R$ may be ignored as considering only the permeate flow $F_H$ is sufficient to capture the flow that is effectively output from the bioreactor through the harvesting and cell separation process. Therefore, the reference to a permeate being present or absent may refer to the auxiliary harvest stream 28C (and derived permeate and retentate - $F_H$ and $F_R$) being present or absent. This permeate $F_H$ may be assumed to comprise medium that has the same composition as the medium in the reactor, but no or few cells. The feed, bleed and permeate flows ($F_F$, $F_B$, $F_H$ and $F_R$) may all be absent, in which case the bioprocess is referred to as an "unfed batch process" or simply "batch process". When a feed flow $F_F$ and a permeate flow $F_H$ are provided, the bioprocess may be referred to as a "perfusion" culture. The process may be referred to as "N-1 perfusion" when the feed flow and permeate flow are present, maintaining the volume of the culture constant in the absence of a bleed flow. The process may be referred to as a "dynamic perfusion" when the feed flow and the permeate and bleed flows are provided such that the bioprocess is operated at (pseudo) steady-state (from a process condition point of view, i.e. maintaining in particular the volume of the culture constant). In both cases, the bioprocess may be referred to as a "continuous" culture. When a feed flow $F_F$ is provided in the absence of output flows (bleed flow and permeate, $F_B$ and $F_H$), the bioprocess may be referred to as a "fed-batch" process. The present disclosure relates in particular to the operation of perfusion flow bioprocesses. The terms "feed", "feed flow", or "feed rate" may be used interchangeably to refer to the flow rate of culture medium in the bioreactor in the feed flow, typically in units of volume per unit of time (such as e.g. litre/hour). The terms "harvest", "harvest flow", "permeate", "permeate rate", "permeate flow" or "harvest rate" may be used interchangeably to refer to the flow rate of culture medium in the permeate, typically in units of volume per unit of time (such as e.g. litre/hour). The terms "bleed", "bleed flow" or "bleed rate" may be used interchangeably to refer to the flow rate of culture medium in the bleed flow, typically in units of volume per unit of time (such as e.g. litre/hour). The feed flow and permeate flow are typically cell free culture media (or essentially cell free, depending on the performance of the cell retention device in the case of the permeate). The bleed flow typically includes culture media and cells. The feed flow may be characterised by one or more parameters including the concentration of one or more substrates therein. The harvest and bleed flows may be characterised by one or more variables including the flow itself (typically in units of volume per time, e.g. litre/hour), and/or the concentration of one or more substrates and/or one or more products therein, typically in units of mass per volume (e.g. g/l). The bleed flow may further be characterised by the viable cell density (also referred to as "viable cell concentration", typically in number of cells per unit of volume - where the viable cell density in the bleed flow is assumed to be the same as the viable cell density in the vessel). The VCD, and concentrations of one or more substrates or products may be assumed to be the same in the bleed flow and in the bioreactor. A viable cell density (the term being used interchangeably with viable cell concentration in the present disclosure) is typically expressed in units of E06/ml, i.e. $10^6$ cells/ml. Additional parameters may be known for each of the flows and/or additional variables may be measured or otherwise determined depending on the particular circumstances of the bioprocess.

A product of a bioprocess (also referred to herein as "biomaterial" or "target biologic") may include a metabolite, a cell, a desired protein, an antibody, an immunoglobulin, a toxin, one or more by-products, a target molecule, or any other type of molecule manufactured using a bioprocess. There may be more than one biomaterial of interest. Products of a bioprocess may have one or more critical quality attributes (CQAs). As used herein, a "critical quality attribute" is any property of a product (including in particular any chemical, physical, biological and microbiological property) that can be defined and measured to characterise the quality of a product. The quality characteristics of a product (in terms of the values of one or more CQAs) may be defined to ensure that the safety and efficacy of a product is maintained within predetermined boundaries. The term "metabolite" refers to any molecule that is consumed or produced by a cell in a bioprocess. Metabolites include in particular nutrients such as e.g. glucose, amino acids etc., by-products such as e.g. lactate and ammonia, desired products such as e.g. recombinant proteins or peptides, complex molecules that participate in biomass production such as e.g. lipids and nucleic acids, as well as any other molecules such as oxygen ($O_2$) that are consumed or produced by the cell. Depending on the particular situation, the same molecule may be considered a nutrient, a by-product or a desired product, and this may even change as a bioprocess is operated. However, all molecules that take part in cellular metabolism (whether as an input or output of reactions performed by the cellular machinery) are referred to herein as "metabolites". In particular, metabolites may include any suitable analyte, including but not limited to: amino acids (e.g., alanine, arginine, aspartic acid, asparagine, cysteine, cysteine, glutamic acid, glutamine, glycine, histidine, hydroxyproline, isoleucine, leucine, lysine, methionine, phenylalanine, proline, serine, threonine, tryptophan, tyrosine, valine, etc.), saccharides (e.g., fucose, galactose, glucose, glucose-1-phosphate, lactose, mannose, raffinose, sucrose, xylose, etc.), organic acids (e.g., acetic acid, butyric and 2-hydroxy- butyric acids, 3-hydroxybutyric acid, citric acid, formic acid, fumaric acid, isovaleric acid, lactic acid, maleic acid, propionic acid, pyruvic acid, succinic acid, etc.), other organic compounds (e.g., acetone, ethanol, pyroglutamic acid, etc.). References to one or more "metabolite concentrations" may include the concentration of one or more metabolites in the culture medium compartment. Unless the context indicates otherwise, metabolite concentrations typically refer to metabolite concentrations in the culture medium. Metabolites that are provided in the culture medium (feed) and that are consumed by the cell's metabolism may be referred to as "substrates". Substrates may include a source of carbon, such as e.g. glucose. Any other molecule or plurality of molecules may be used as substrates depending on the particular bioprocess.

**Advanced Control of Perfusion Flow Bioprocesses**

The present disclosure relates to an advanced control strategy for cell culture processes, in particular for perfusion operating modes. As explained above, perfusion refers to the use of media exchange where fresh media is continuously added while spent media is continuously removed to maintain a constant working volume. Cells are retained in the cell culture through a separation device such as an alternating tangential flow (ATF) or tangential flow filtration (TFF), where cell free spent media is removed through a harvest stream (permeate), cells are retained via a membrane filter and returned to the bioreactor. In addition to the feed and permeate streams, a bleed stream is typically included to maintain a desired viable cell density (VCD or viable cell concentration, VCC). The bleed contains material contents identical to that in the working volume of the bioreactor. These three flow streams (feed, permeate and bleed) may be controlled to maintain a desired media exchange rate, supply of sufficient nutrients, cell density and working volume. A media exchange rate may be defined as the total volume of medium that flows into the bioreactor or out of the bioreactor per unit of time. The total volume of medium that flows into the bioreactor per unit of time is typically equal to the total volume of medium that flows out of the bioreactor per unit of time.

In a simple implementation, three independent single loop controllers (such as PID - proportional integral derivative controller) could be used to control each of the feed, harvest and bleed flows. Control of the feed flow may be used to maintain the media exchange rate (constant target or a ratio of the VCD), or control the concentration of a substrate. Control of the harvest may be used to control the working volume. Control of the bleed flow may be used to control the VCD. In some cases the harvest and feed objectives may be reversed, where harvest is used to maintain the media exchange rate and feed to control volume. The present inventors have recognized that in this type of configuration these three control loops interact with each other resulting in oscillatory type behavior, destabilizing the process. This oscillatory behavior may be exacerbated by the pulse type action that is typical for ATF separation devices .

By contrast, according to the present disclosure, state space models are used within a model predictive control (MPC) structure to predict the future trajectory of at least one of the parameters to be controlled (referred to as control variable(s) or CV, such as e.g. media exchange or target substrate concentration, VCD and volume) to determine optimal adjustment to at least one of the manipulated variables (MV, e.g. one or more of the three flow streams mentioned above) in order to maintain the CV at their targets at a specified future time point. In other words, one or more control loops are provided to control the concentration of a substrate, the working volume and the VCD (either jointly or independently), by manipulating the feed flow, the permeate flow and the bleed flow, at least one of which uses a model predictive controller. For example, a control loop with a model predictive controller may be used to control the substrate concentration by manipulating the feed flow, and two independent P/PI/PID controllers may be used to control the working volume by manipulating the permeate flow, and the VCD by manipulating the bleed flow, respectively. As another example, all of the substrate concentration, working volume and VCD may be controlled jointly by manipulating the feed, bleed and permeate flows through a model predictive controller that jointly models all of these variables. As another example, the substrate concentration and VCD may be controlled jointly by manipulating the feed, bleed and permeate flows through a model predictive controller that jointly models all of these variables.

Further, the model predictive controller uses an objective function that prioritizes minimization of the adjustment to the MV over minimization of the error in CV. This controls the process in a manner that is more stable than control methods of the prior art and also more stable than a simpler approach using three independent conventional controllers (i.e. controllers that do not include a dynamic model of the process to be controlled, such as e.g. simple P, PI, PD, or PID controllers). Additionally, the control method may include a self-adaptive state space model update. This may further improve the stability of the control method, especially over long bioprocess runs where e.g. changes in physiology of the cells may occur. This may be particularly useful to improve stability of the controlled process when the substrate and VCD are not jointly controlled by a MCP, although in such cases the self-adaptive state space model (i.e. automated state space model update) may further improve stability. In embodiments, the VCD target may be modified to control the total viable cells instead of the VCD. This may further improve the stability of the process by preventing adjustments in volumes to be used to control the VCD. In embodiments, the method may further comprise using a control objective that maintains a target permeate flow rate. This may reduce unwanted variations in permeate flow rates that may cause problems for downstream processes, thereby enabling integrated upstream/downstream continuous operation.

**Figure 2** illustrates schematically the logic flow in an exemplary control process according to the disclosure. At each iteration of the control method, a set of parameters of the process are measured or otherwise determined at step 200a. In the illustrated embodiment, the following parameters from the process are obtained during operation of the process: Viable cell density (VCD also called viable cell concentration, VCC), Volume, feed flow rate, permeate flow rate and bleed flow rate, and optionally also the concentration of a substrate such as e.g. glucose. These may be measured or set by effectors (e.g. valves controlling a flow rate). In embodiments, one or more of these parameters may not be directly measured but rather calculated from other measurements. For example, the volume may be calculated from a weight from a scale. As another example, one or more flows may be calculated from a rate of change of a weight scale. One or more of these parameters may be measured continuously or close to continuously (e.g. a very frequent intervals), or at least at every iteration of the control method (step 200a). This may be performed for example using on-line sensors that measure the one or more parameters or determine them from other measurements performed on medium or flows during operation of the bioprocess. Additionally, one of more of these parameters may optionally be measured at low frequency, such as less frequently than at each iteration of the control method (step 200b). This may be performed for example using off-line protocols, such as e.g. by obtaining a sample of medium and analysing it in a laboratory. Thus, a first set of parameters may be measured at a frequency or set of frequencies that are higher than a frequency or set of frequencies at which a second set of parameters is measured. The first and second set may overlap, i.e. the same parameter may be measured both at a first (higher) frequency (e.g. using an on-line sensor) and at a second (lower) frequency (e.g. by off-line analysis). This may be useful for example when the off-line analysis provides a measurement that is more accurate than the on-line measurement. Note that both measurements 200a and measurements 200b may be collected at each iteration of the control method, in which case measurements 200a may comprise for each measured variable a plurality of measurements that relate to a plurality of time points and measurements 200b may comprise for each measured variable one or more measurements that relate to one or more time points, the one or more time points being fewer time points than the plurality of time points (i.e. less frequent measurements). The measurements 200a and 200b provide the current (or latest measured) value of one or more controlled variables and one or more manipulated variables of the system.

The measurements 200a and optionally 200b are provided to a state observer 210. The state observer 210 produces estimates of one or more state variables of the bioprocess using measurements 200a and optionally 200b. For example, the state observer 210 may be used to provide estimates of controlled variables (in the illustrated embodiment these are the VCD (or total cells) and substrate concentration, although these may include any of VCD/total cells, substrate concentration, media exchange rate, and volume), based on the measurements 200a, 200b and a state space model. The functioning of the state observer will be described in more detail below. The estimates of the state observer 210 (in the illustrated embodiment, the VCD and substrate concentration) as well as one or more of the measured variables 200a (in the illustrated embodiment, the feed, harvest and bleed flows, which are the current values of the manipulated variables of the process) are provided to an advanced control method 220, which in the illustrated embodiment is a model predictive controller (MPC).

The controller 220 may further take as input one or more parameters 230. The one or more parameters 230 may include operational targets (e.g. setpoints and optionally high and/or low thresholds for one or more controlled variables, in the illustrated embodiments these include targets for the VCD/total cells, the volume, and the media exchange rate, and optionally targets for the substrate (this may replace or complement a media exchange rate target) and/or the permeate flow). The one or more parameters 230 may include controller parameters (referred to as "controller tuning" on Figure 2) which determine e.g. how the controller balances the different objectives (e.g. the weights of a control objective as described further below). Using these inputs, the controller 220 identifies an optimal control action (i.e. changes to one or more manipulated variables). In the illustrated embodiment these are new targets for feed, harvest and bleed flow rates. These are provided to a distributed control unit 240, which controls effectors to implement these targets. The distributed control unit also provides the high frequency measurements 200a that are used at the next iteration of the control method. The distributed control unit may be in operable connection with one or more actuators (also referred to as "effectors", e.g. pumps, valves, etc.) and/or one or more sensors (e.g. on-line VCD sensor, volume sensor, etc.) for this purpose. Such control units are known in the art and a detailed description of these is beyond the scope of the present disclosure. The control method may be implemented using an initialization phase, in which the controller 220 is not used for one or more iterations of the control method. This enables the state observer 210 to be initialized. For example, an initial period may be used to collect enough measurements to obtain good predictions from a Kalman Filter. Typically, only a few measurements may be sufficient to have good Kalman filter estimates, such as e.g. 5 or 6 iterations (measurements). Instead or in addition to this, the initial period may be set for a period such that the cells reach a density that is such that a calculated cell specific perfusion rate (CSPR) is above a minimum feed rate (where a minimum feed rate may be set to ensure that there is a feed flow). Thereafter, the feed may be set based on expected cell density and/or desired substrate concentration. For example, at a first iteration, a first set of measurements 200a (and optionally 200b) may be used together with initial (user defined) values for one or more states of the state space model that cannot be directly measured. As explained above and further below, the state space model may be used both by the state observer 210 and by the model predictive controller 220. These values are used to integrate the state space model to predict the value of state space variables until the next set of measurements 200a (and optionally 200b) is received. At the next iteration, the state space observer (e.g. a Kalman filter) is used to calculate new values for the state space variables, using the latest set of measurements 200a (and optionally 200b). These are again used to integrate the state space model to predict the value of state space variables until the next set of measurements 200a is received. This may be repeated for one or more iterations together forming the initialization period. This results in a number of estimates of the state space variables (also referred to as "system states"), which can be used by the state space observer (e.g. a Kalman Filter) to obtained improved predictions of state space variables in future iterations. After the initialization phase, the state space observer (e.g. a Kalman filter) is still used to calculate new values for the state space variables at every iteration, using the latest set of measurements 200a (and optionally 200b), but this is then used by the model predictive controller to determine a first control action for one or more manipulated variables. The model predictive controller uses the estimates of the state space variables from the state space observer to integrate the state space model to determine an control action to be taken which is optimal over a prediction horizon. The integration may be performed at a certain frequency until a new set of measurements 200a (and optionally 200b) is obtained, at which point anew iteration starts, and new state space variable estimates are obtained by the state observer 210.

**Model Predictive Controller**

In the context of the present disclosure, a model predictive control (MPC) is a process control method that involves the prediction of the future trajectory of parameters to be controlled (e.g. media exchange rate or substrate concentration, VCD or total viable cell, and volume) in order to determine optimal adjustments to be made to manipulated variables (e.g. flow streams) to maintain the controlled variable at desired targets at a specified future timepoint. This uses a model that represents the behaviour of the process, referred to herein as "state space model" (described below). Such models can predict the change in dependent variables (including controlled variables) of the modelled system that will be caused by changes in independent variables (including the manipulated variables) of the modelled system. MPC control involves obtaining measurements indicative of the current state of the bioprocess at time t, then using an internal dynamic model of the process to simulate one or more states of the system over a time horizon. These are used as variables of a cost function over the time horizon [t, t+T] (also referred to herein as "control objective", as further described below) and an optimisation algorithm is used to identify a control input strategy that minimises the cost function over the time horizon [t, t+T]. The behaviour of the system is typically predicted over multiple time points included in the prediction horizon, such as e.g. time points between t and t+T separated by an interval $t_1$ (e.g. [t, t+t$_1$, t+2t$_1$, t+3t$_1$,..., t+T]). Typically, the first step of the control input strategy (e.g. value of manipulated variable(s) at t+t$_1$) is implemented (or any step corresponding to time points between measurements of the state of the bioprocess), and the calculation is repeated every time a new set of measurements of the current state of the bioprocess is obtained. In particular, the calculation may be conducted every time a new set of one or more on-line measurements of the current state of the bioprocess is obtained. At every calculation step (also referred to herein as "iteration of the control loop", or simply "iteration"), the results of the calculation may include the values of one or more manipulated variables (e.g. feed flow rate) and the values of one or more controlled variables (e.g. glucose concentration), that are optimal according to a control objective comprising set points for the controlled variables over the time horizon. The time horizon [t, t+T] has a length T that may be selected to be sufficiently long to capture complex dynamics that are expected to be present, in order to ensure convergence to the optimum solution. For example, the length T of the time horizon may be selected to extend to the time necessary for the system to reach a steady state. Instead or in addition to this, the length of the prediction horizon may be determined using one or more factors selected from: the reactor residence time, availability of model-base estimates as a safety feature in case of sensor downtime or failure, and computational resources may be

**Control Objective**

A control solution is found by finding a set of adjustments in manipulated variables that minimize a control objective. A control objective is a mathematics expression quantifying the desirability of operation. Various forms may be used. A generic example of a quadratic control objective may be in the form of:

$$J = \sum_{i=1}^{N} w_{x_i} (r_i - x_i)^2 + \sum_{i=1}^{N} w_{u_i} \Delta u_i^2$$

where J is the cost function to be minimized, $x_i$ is the i$^{th}$ controlled variable (e.g. media exchange rate, substrate concentration, VCD, total viable cell, volume), $r_i$ is the set point for the i$^{th}$ controlled variable, $u_i$ is the i$^{th}$ manipulated variable (e.g. feed, harvest, bleed flows), and w are weighing coefficients that reflect the relative importance of the controlled variables ($w_{xi}$) and penalize large changes in manipulated variables ($w_{ui}$). Note that in each of the control objectives described herein, references to values of controlled and manipulated variables may refer to those values at a plurality of time points (e.g. respectively a prediction of the states of the system over a prediction horizon and the values of the manipulated variables in a corresponding control strategy). For example, in the equation above, each term of the sums may be repeated for *n* time steps (e.g. corresponding to n iterations of a control loop), where the values of the system's internal state at each of these time steps is predicted using a state space model (and optionally also a state space observer) as described further herein. Depending on the control strategy used, only the first control step ($\Delta u_i$ corresponding to the first time step in the prediction horizon) may be implemented, and the simulation and optimization may be repeated with new values of the measured variables of the system at the next iteration of the control method.

According to the present disclosure, the control objective minimizes perturbations to the process by minimizing adjustment to the manipulated variables, while simultaneously maintaining the controlled variables in proximity of their targets. An example control objective takes the form in equation (1).

$$\min f_{obj} = p_x(\widehat{x_v} - x_{v,set})^2 + p_V(\hat{V} - V_{set})^2 + p_s(\hat{s} - s_{set})^2 + p_m(\hat{m} - m_{set})^2$$
$$+ p_f \Delta F_f + p_h \Delta F_h + p_b \Delta F_b$$

$$(1)$$

Where

$$\Delta F_f = |F_{f,new} - F_{f,current}| \tag{2}$$

$$\Delta F_h = |F_{h,new} - F_{h,current}| \tag{3}$$

$$\Delta F_b = |F_{b,new} - F_{b,current}| \tag{4}$$

or

$$\Delta F_f = |F_{f,new} - F_{f,current}|^2 \tag{5a}$$

$$\Delta F_h = |F_{h,new} - F_{h,current}|^2 \tag{6a}$$

$$\Delta F_b = |F_{b,new} - F_{b,current}|^2 \tag{7a}$$

In equations (1)-(4), $f_{obj}$ is the objective function to be minimized; $\widehat{x_v}$ is the estimated future VCD; $x_{v,set}$ is the desired VCD; $p_x$ is the penalty for deviations in VCD; $\hat{V}$ is the estimated future working volume of the bioreactor; $V_{set}$ is the desired working volume; $p_v$ is the penalty for deviations in working volume; $\hat{s}$ is the estimated future substrate concentration; $s_{set}$ is the desired substrate concentration; $p_s$ is the penalty for deviations in substrate concentration; $\hat{m}$ is the estimated future media exchange rate (either a constant flow target or a ratio of the total cells); $m_{set}$ is the desired media exchange rate; $p_m$ is the penalty for deviations in media exchange rate; $F_{h,set}$ is the desired permeate flow rate; $p_m$ is the penalty for deviations in permeate flow rate; $F_f$ is the feed flow rate; $F_h$ is the permeate flow rate; $F_b$ is the bleed flow rate; $\Delta F_f$ is the difference between the current feed flow rate and a proposed new feed flow rate (proposed change in feed flow rate); $\Delta F_h$ is the difference between the current permeate flow rate and a proposed new permeate flow rate (proposed change in permeate flow rate; and $\Delta F_b$ is the difference between the current bleed flow rate and a proposed new bleed flow rate (proposed change in bleed flow rate).

A state space model is used to predict future values of the controlled variables (CV), which in this example include $\widehat{x_v}$, $\hat{V}$, $\hat{s}$, and $\hat{m}$. Examples of state space models are provided below. Future value of the CV are dependent on the feed, harvest and bleed flows. In the objective function (1), the manipulated variables (MV) are $\Delta F_f$, $\Delta F_h$ and $\Delta F_b$. In embodiments, the substrate and/or media exchange terms (i.e. $p_s(\hat{s} - s_{set})^2$ and $p_m(\hat{m} - m_{set})^2$) may not be included in the control objective. In embodiments where both terms are excluded, the user may set either the feed or permeate flow rates to a specified value.

The media exchange rate can be expressed in one of three forms as in equation (8a)-(5c).

$$\hat{m} = F_f \tag{8a}$$

$$\hat{m} = \frac{F_f}{V_{set}} \tag{8a}$$

$$\hat{m} = F_f x_v V_{set} \tag{8a}$$

In the first media exchange rate case, shown in equation (8a), the target is a simple constant value. In the second case (8a), the target is a ratio of the feed flow to the target working volume. The third case (8a), the exchange rate is scaled based on the number of viable cells. The third case may be referred to as a cell specific perfusion rate (CSPR).

In embodiments, the control objective may include a term that aims to maintain a total viable cell target instead of a term that aims to maintain a viable cell density target. The objective described in equation (1) includes a penalty for deviations in the VCD from its target. Maintaining a desired cell concentration is typical in perfusion processes. Modifying the objective to maintain a desired number of cells in the bioreactor decouples the control of cells from that of the volume, further improving the stability of the controller. This prevents the controller from increasing the volume, hence diluting the cell concentration, if the VCD is above its target or reducing the volume if the VCD is below its target. The controller should instead select control action that that maintains a desired number of total cells. Thus, an enhancement to the control objective from equation (1) is shown in equation (6).

$$\min f_{obj} = p_x\left(\widehat{x_v}\hat{V} - x_{v,set}V_{set}\right)^2 + p_V\left(\hat{V} - V_{set}\right)^2 + p_s(\hat{s} - s_{set})^2$$
$$+ p_m(\hat{m} - m_{set})^2 \tag{6}$$
$$+ p_f \Delta F_f + p_h \Delta F_h + p_b \Delta F_b$$

In equation (6), all the terms have the same meaning as above except that $\widehat{x_v}$ is the estimated future total cells, $x_{v,set}$ is the desired number of total cells, and $p_x$ is the penalty for deviations in the quantity of total viable cells. Note that when simultaneously controlling the volume to a predetermined target and the total number of cells to a predetermined target, it is possible to control the viable cell density to a predetermined target. Thus, a user may simply specify a target volume and a target VCD even when using embodiments that maintain a total number of cells, and these may be used in the objective function to obtain a term that penalizes deviation from a target total number of cells (e.g. equation (6)). However, the use of a control objective which includes a term that penalizes deviation from a predetermined target volume as well as a term that penalizes deviation from a predetermined target number of cells ensures that the controller does not optimize the objective function by manipulating the volume to reach a target viable cell density if other actions can be taken. Indeed, in the absence of a term that penalizes deviation from a predetermined target number of cells, a controller may "sacrifice" the volume regulation by selecting a control action that reduces the volume to reach a target viable cell density. Such a "simplistic" control action is unlikely to be selected when using a control objective that includes a term penalizing deviation from a target total number of cells instead of a term penalizing deviation from a target viable cell density as it would not achieve the objective of bring the total number of cells closer to its target. This is by contrast with the simplest objective of maintaining a target viable cell density, which can be achieved by reducing the volume.

In embodiments, the control objective is expanded to include additional penalties to the objective function that penalize deviations from set minimum and maximum values of specific setpoints (also referred to as "thresholds" or "setpoint thresholds"), as shown in equation (7). This may further improve process stability.

$$\min f_{exp} = f_{obj} + p_{x,hi}\left(max\left(\widehat{x_v} - x_{v,hi}, 0\right)\right)^2 + p_{x,lo}\left(min\left(\widehat{x_v} - x_{v,lo}, 0\right)\right)^2$$
$$+ p_{V,hi}\left(max\left(\hat{V} - V_{hi}, 0\right)\right)^2 + p_{V,lo}\left(min\left(\hat{V} - V_{lo}, 0\right)\right)^2$$
$$+ p_{s,hi}\left(max(\hat{s} - s_{hi}, 0)\right)^2 + p_{s,lo}\left(min(\hat{s} - s_{lo}, 0)\right)^2 \tag{7}$$
$$+ p_{m,hi}\left(max(\hat{m} - m_{hi}, 0)\right)^2 + p_{m,lo}\left(min(\hat{m} - m_{lo}, 0)\right)^2$$

In equation (7), $f_{obj}$ is an objective function to be minimized such as e.g. that provided in equation (6); $f_{exp}$ is the expanded objective function including minimum and maximum thresholds; $\widehat{x_v}$ is the estimated future total cells or VCD; $x_{v,hi}$, $x_{v,lo}$ are the total cells or VCD low and high thresholds; $p_{x,hi}$, $p_{x,lo}$ are the penalties for deviations in total cells or viable cell concentration below/above the low and high thresholds; $\hat{V}$ is the estimated future working volume of the bioreactor; $V_{hi}$, $V_{lo}$ are the low and high thresholds for desired working volume; $p_{V,hi}$, $p_{V,lo}$ are the penalties for deviations in working volume below/above the low and high thresholds; $\hat{s}$ is the estimated future substrate concentration; $s_{hi}$, $s_{lo}$ are the low and high thresholds for desired substrate concentration; $p_{s,hi}$, $p_{s,lo}$ are the penalties for deviations in substrate concentration below/above the low and high thresholds for desired substrate concentration; $\hat{m}$ is the estimated future media exchange rate (either a constant flow target or a ratio of the total cells); $m_{hi}$, $m_{lo}$ are the low and high thresholds for desired media exchange rate; $p_{m,hi}$, $p_{m,lo}$ are the penalties for deviations in media exchange rate below/above the low and high thresholds. Any of the terms in equation (7) may be omitted, for example where there is no desired permeate flow rate, or where there is no low or high threshold for any of the variables above.

The controller may be tuned (i.e. the parameters of equation 7 or any equivalent equation may be adjusted) such that the penalties for the high and low thresholds is greater, preferably much greater, than the penalty of the deviation from desired target setting. As an example setting $p_V$ to a much lower value than $p_{V,hi}$ and $p_{V,lo}$ will configure the controller to make minimal control actions unless it is predicted the working volume will exceed a high or low threshold. Configuring the controller tuning where the high and low penalties are sufficiently large converts the high and low thresholds into soft constraints. This approach may be useful for example for the control of the volume in the bioreactor, to enable the control process to adjust the volume as necessary to maintain other controlled variables at their set point, while preventing such adjustments to put the volume outside of a range that is safe (e.g. in terms of risk of overflow of the vessel). This combination of allowing flexible adjustments to maintain setpoints while including stronger constraints advantageously balances the objectives of stability of the bioprocess and adherence to constraints that may be set by physical or biological properties of the bioprocess. In the extreme setting $p_V = 0$ will create a deadband where no control action is taken if $\hat{V}$ is predicted to remain between the high and low thresholds. A secondary benefit of threshold is the ability to provide multi-mode type of action. For example there are often minimum specifications for media exchange rate and glucose levels. The controller may be tuned to target a desired glucose concentration while at the same time assure maintenance of a minimum media exchange rate.

In embodiments, the control objective may include a term that aims to maintain a consistent harvest rate. It is common that perfusion cell culture processes are connected to downstream purification systems to create an integrated continuous system. The input to the downstream unit is the harvest stream. Efficient continuous operation of the downstream process therefore requires a consistent permeate flow rate. Maintaining a consistent permeate flow rate can be achieved by adding the terms in equation (9) to the control objective, where the term $f_{obj}$ can be replaced by $f_{exp}$.

$$\min f_h = f_{obj} + p_h\left(F_h - F_{h,set}\right)^2 + p_{h,hi}\left(max\left(F_h - F_{h,hi}, 0\right)\right)^2 + p_{h,lo}\left(min\left(F_h - F_{h,lo}, 0\right)\right)^2 \qquad (9)$$

In equation (8), $f_{obj}$ is an objective function to be minimized such as e.g. that provided in equation (6) or equation (7), $F_{h,set}$, $F_{h,hi}$, $F_{h,lo}$ are the desired permeate flow rate and low and high thresholds for the desired permeate flow rate, and $p_h$, $p_{h,hi}$, $p_{h,lo}$ are the penalties for deviations in permeate flow rate from target and below/above the low and high thresholds. Any of the harvest terms in equation (8) may be present or absent, in some embodiments. Thus, an objective function according to any embodiment described herein may comprise any one or more of (such as, in particular, one, two or all of): a term that penalizes deviation from a predetermined target for the permeate flow ($F_{h,set}$), a term that penalizes deviation from a predetermined high threshold for the permeate flow ($F_{h,hi}$), and a term that penalizes deviation from a predetermined low threshold for the permeate flow ($F_{h,lo}$).

In any of equations (1), (6), (7) and (8) above, any of the controlled and manipulated variables may be omitted, for example where the controller is used to control a subset of the controlled variables using a subset of the manipulated variables. For example, a controller that controls the substrate concentration (controlled variable) through control of the feed flow (manipulated variable) may use a control objective that only contains terms for the substrate and feed flows. Further, any of the controlled variables may be associated with a term that penalizes deviation from a target and one, both or none of terms that penalizes deviations from a low threshold and a high threshold. For example, one or more of the controlled variables may be associated with a term that penalizes deviation from a corresponding target, but no term that penalizes deviations from high/low thresholds. Another one or more of the controlled variables may be associated with a term that penalizes deviation from a corresponding target, and a term that penalizes deviation from a corresponding high threshold or a low threshold. Yet another one or more of the controlled variables may be associated with a term that penalizes deviation from a corresponding target, a term that penalizes deviation from a corresponding high threshold, and a term that penalizes deviation from a corresponding low threshold.

The control objective may balance a plurality of objectives, not all of which can be met at the same time. For example, when controlling 3 manipulated variables (e.g. feed, harvest and bleed flow rates), it is not possible to identify values of the manipulated variables that enable to simultaneously bring all the controlled variables (e.g. the volume, VCD and substrate concentration) to any arbitrary target values. Thus, the objective function may balance respective control objectives to prioritize some of the control objectives over others, for example by assigning weights to the various control objectives. This is achieved in the equations above by setting the relative values of the coefficients p to reflect the order of priority of the respective control objectives. In embodiments, the use of relatively small adjustments may be more important than whether a particular variable is close to its target, and one or more of $p_x$, $p_v$, $p_s$ and $p_m$ may accordingly be set to values that are lower than one or more of (e.g. all of) $p_F$, $p_H$, and $p_B$. The controller may be tuned (i.e. the parameters of equations (1), (6), (7) and (8) or any equivalent equation may be adjusted) such that the penalties for the adjustment to the manipulated variables is equal to or greater than the penalty of the deviation from desired target setting. As an example setting $p_S$ to a value that is equal to or lower than the value for $p_F$ will configure the controller to prioritize making small adjustments to the feed flow rate over reaching the substrate target. The actual value of each of the terms does of course still depend on the value of the deviation between target and measured/predicted substrate concentration and the proposed step change to the manipulated variable(s), such that larger step changes to manipulated variables will be selected by the controller when the substrate concentration is further away from its target then when the substrate concentration is closer to its target. The values of the respective weights may be set to achieve this objective using a variety of approaches. In embodiments, the control method may be implemented in a test process and/or in a simulated process, and the behavior of the system may be compared when using a plurality of candidate sets of values of the weights in the objective function. One of the candidate sets of values may be selected for subsequent use, or to obtain a set of values for subsequent use. For example, the behavior of the system in terms of one or more of: ability of the control method to bring controlled variables sufficiently close to their targets (where a controlled variable may be considered to be sufficiently close to its target when it is within a predetermined range around its target for a predetermined portion of the operation of the bioprocess), ability of the control method to avoid oscillatory behaviors in the setting of one or more manipulated variables, ability of the control method to avoid overcompensating behaviors in the setting of one or more manipulated variables. Overcompensating behaviors may be identified as local minima or maxima in the trajectory of a manipulated variable, the local maxima/minima having one or more predetermined characteristics. The one or more characteristics may be for example the a height to width ratio of the local extremum in manipulated variable being over a predetermined value. The height of a local extremum of a manipulated variable may be the highest step change between the value of the extremum and the closest value at which the manipulated variable is not increasing/decreasing. The width of a local extremum of a manipulated variable may be the time between when the first points where the manipulated variable is not increasing/decreasing, around a local extremum value. Preferably, the values of the respective weights are adjusted to penalize making large changes to the manipulated variables, as this may destabilize other parts of the system. In general, penalizing changes to the manipulated variables tunes the controller to slowly bring the control variables to their targets. Given that for cell culture processes maintaining a stable process is prioritized over removing small deviations in the controlled variables from their targets, the controller therefore operates to minimize changes to MV subject to maintaining the CV 'close' to their targets (rather than the more usual objective of minimizing differences between CV and their targets). Model predictive controllers using control objectives that penalize changes to the manipulated variables are unlikely to display oscillatory behaviors, unless there is a mismatch between the model and the system. Thus, as described further herein, it is beneficial for the controller to further include the feature of self-updating of model coefficients, such as e.g. automated updating of the specific consumption rate of the selected nutrient (e.g. glucose) and the specific growth rate of the cells, as this further enhances the stability of the control method. The present inventors have found the models described herein to be very stable and transferable to multiple different bioprocesses with limited changes. Thus, the parameters in any of equations (1), (6), (7) and (8), and in particular the weights in these equations, may be set as described above for one bioprocess, and re-used for a different bioprocess. Further, a combination of setpoints for the controlled variables may be set within operating windows that have been identified for the system (such as e.g. ranges of values of controlled variables that can be achieved within the system and/or that can be achieved with a stable behavior in the system). Within these operating windows, the relative values of the coefficients p may be adapted by a user depending on their particular requirements, such as e.g. a user may wish to keep glucose constant at all means, even if this means a deviation on another parameter. In such cases, the coefficient $p_s$ met be set to a high value compared to the other weighing coefficients. The operating windows may be identified by simulating the behavior of the system.

The value of the weights in equations (1), (6), (7) and (8) are typically set to respective values that are constant during operation of the bioprocess. In embodiments, the value of one or more of the weights in any of equations (1), (6), (7) and (8) may be set to a first respective value at a first time during operation of the bioprocess, and a second (or further) respective value at a second (or further) time during operation of the bioprocess. In other words, the respective importance of the control objectives set out in equations (1), (6), (7) and (8) may vary during operation of the bioprocess. This may be advantageous for example when the state of the cells and/or the state of one or more parts of the bioreactor system may vary over time. For example, different cell culture phases may be controlled using differently tuned control objectives (e.g. a process may comprise a growth phase followed by a stationary perfusion phase, and these phases may be optimized individually and/or follow a different trajectory of setpoints). Similarly, in embodiments, the value of one or more of the targets (setpoints) and/or high/low thresholds in any of these equations may be set to a first respective value at a first time during operation of the bioprocess, and a second (or further) respective value at a second (or further) time during operation of the bioprocess. In other words, the values of the setpoints and/or thresholds used in the control objectives set out in equations (1), (6), (7) and (8) may vary during operation of the bioprocess. This may be advantageous for example when the state of the cells and/or the state of one or more parts of the bioreactor system may vary over time. For example, when the cell culture is expected to go through one or more growth phases during operation of the bioprocess, the target VCD may be adapted accordingly. For example, a substrate concentration target may be set to a higher level in a first phase (e.g. growth phase, in order to promote growth), then to a lower level in a second phase (e.g. steady-state culture). As another example, the target VCD may be set to a first level in a first growth phase (e.g. until a target VCD is reached), then to a different level in a later growth phase, to reflect the natural tendency of the health of the cell culture to degrade over time.

Any of the terms of equations (1), (6), (7) and (8) may be removed to reflect the controlled and/or manipulated variables that are being controlled in the same control loop. Other controlled variables may be controlled using other manipulated variables in one or more independent control loops. Thus, the method may comprise using a plurality of independent control loops each controlling a different one or more of the controlled variables using a different one or more of the manipulated variables. At least one of the control loops may use a model predictive controller as described herein. Any one or more additional control loops may use standard controllers as known in the art, such as e.g. a proportional (P) controller, a proportional derivative (PD) controller, or a proportional integral derivative (PID) controller. Thus, in embodiments, a model predictive controller may be used to control the value of a single controlled variable using a single manipulated variable. For example, a model predictive controller may be used to control the concentration of a substrate using the feed flow rate as a manipulated variable. Such a control method may use a control objective of the form given in equation (1a) or (7a):

$$\min f_{obj} = p_s(\hat{s} - s_{set})^2 + p_f \Delta F_f \tag{1a}$$

$$\min f_{exp} = f_{obj} + p_{s,hi}\big(max(\hat{s} - s_{hi}, 0)\big)^2 + p_{s,lo}\big(min(\hat{s} - s_{lo}, 0)\big)^2 \tag{7a}$$

where equations (1a) and (7a) can also be expressed as equation (1b) and (7b), respectively:

$$\min f_{obj} = \sum_{j=1}^{n} \big(\widehat{s_{k+j}} - s_{set,k+j}\big)^2 p_s + \sum_{j=0}^{m-1} p_f \Delta F_f \tag{1b}$$

$$\min f_{obj} = \sum_{j=1}^{n} \big(\widehat{s_{k+j}} - s_{set,k+j}\big)^2 p_s$$

$$+ \sum_{j=0}^{m-1} p_f \Delta F_f + \sum_{j=1}^{n} \big(max \, (\widehat{s_{k+j}} - s_{hi}, 0)\big)^2 p_{s,hi} \tag{7b}$$

$$+ \sum_{j=1}^{n} \big(min \, (\widehat{s_{k+j}} - s_{lo}, 0)\big)^2 p_{s,lo}$$

where k is the current iteration and j is the number of steps in a prediction horizon from k to n (for state variables) or m (for controller outputs). In such embodiments, the volume and viable cell density may be controlled respectively using the harvest and bleed rates, through independent control loops with e.g. PID controllers. In other embodiments, a model predictive controller may be used to control the value of two controlled variable using two or more manipulated variables. Thus, as another example, a model predictive controller may be used to control the value of two controlled variable, such as the media exchange rate and concentration of a substrate, using two manipulated variables, such as e.g. the feed flow rate and the bleed flow rate, or three manipulated variables, such as e.g. the feed, harvest and bleed flow rates. In such embodiments, the control method may use a control objective of the form given in equation (1c) or (7c):

$$\min f_{obj} = + p_s(\hat{s} - s_{set})^2 + p_m(\hat{m} - m_{set})^2 + p_f \Delta F_f + p_b \Delta F_b \qquad (1c)$$

$$\min f_{exp} = f_{obj} + p_{s,hi}\big(max(\hat{s} - s_{hi}, 0)\big)^2 + p_{s,lo}\big(min(\hat{s} - s_{lo}, 0)\big)^2 + p_{m,hi}\big(max(\hat{m} - m_{hi}, 0)\big)^2 + p_{m,lo}\big(min(\hat{m} - m_{lo}, 0)\big)^2 \qquad (7c)$$

This may be useful for example when it is desirable to have a minimum media exchange rate that is maintained, while at the same time keeping a substrate (e.g. glucose) concentration at a predetermined setpoint. Thus, equation (7c) may be used without a term for the maximum media exchange rate and/or without a term for a set medium exchange rate in $f_{obj}$ defined in equation (1c).

As another example, the viable cell density, perfusion rate (media exchange rate), and volume may be controlled using the feed flow, harvest flow and bleed flow rates as manipulated variables. In such embodiments, the control method may use a control objective of the form given in equation (1d), (6d) or (7d):

$$\min f_{obj} = p_x(\widehat{x_v} - x_{v,set})^2 + p_V(\hat{V} - V_{set})^2 + p_m(\hat{m} - m_{set})^2 + p_f \Delta F_f + p_h \Delta F_h + p_b \Delta F_b$$
$$(1d)$$

$$\min f_{obj} = p_x(\widehat{x_v}\hat{V} - x_{v,set}V_{set})^2 + p_V(\hat{V} - V_{set})^2 + p_m(\hat{m} - m_{set})^2 + p_f \Delta F_f + p_h \Delta F_h + p_b \Delta F_b$$
$$(6d)$$

$$\min f_{exp} = f_{obj} + p_{x,hi}\left(max(\widehat{x_v} - x_{v,hi}, 0)\right)^2 + p_{x,lo}\left(min(\widehat{x_v} - x_{v,lo}, 0)\right)^2 + p_{V,hi}\left(max(\hat{V} - V_{hi}, 0)\right)^2 + p_{V,lo}\left(min(\hat{V} - V_{lo}, 0)\right)^2 + p_{m,hi}\big(max(\hat{m} - m_{hi}, 0)\big)^2 + p_{m,lo}\big(min(\hat{m} - m_{lo}, 0)\big)^2$$
$$(7d)$$

Note that this model can be extended to include e.g. substrate control (as per equations (1), (6), (7)) or reduced for example by removing the media exchange rate term, for example if there is no target media exchange rate.

As yet another example, a substrate concentration (e.g. glucose concentration) may be controlled simultaneously with a viable cell density. In such embodiments, the control method may use a control objective of the form given in equation (1e), (6e) or (7e):

$$\min f_{obj} = p_x(\widehat{x_v}\hat{V} - x_{v,set}V_{set})^2 + p_s(\hat{s} - s_{set})^2 + p_f \Delta F_f + p_h \Delta F_h + p_b \Delta F_b \qquad (1e)$$

$$\min f_{obj} = p_x(\widehat{x_v}\hat{V} - x_{v,set}V_{set})^2 + p_s(\hat{s} - s_{set})^2 + p_f \Delta F_f + p_h \Delta F_h + p_b \Delta F_b \qquad (6e)$$

$$\min f_{exp} = f_{obj} + p_{x,hi}\left(max(\widehat{x_v} - x_{v,hi}, 0)\right)^2 + p_{x,lo}\left(min(\widehat{x_v} - x_{v,lo}, 0)\right)^2 + p_{s,hi}\big(max(\hat{s} - s_{hi}, 0)\big)^2 + p_{s,lo}\big(min(\hat{s} - s_{lo}, 0)\big)^2 \qquad (7e)$$

Combined control of substrate concentration and viable cell density (whether including or not one or more additional control objectives related to volume control, media exchange rate control, and permeate flow rate control) may advantageously reduce the reliance on measurements of the substrate concentration and viable cell density. In particular, a control method that controls both the substrate concentration and the viable cell density (or total number of cells) using a model predictive controller that jointly predicts VCD and substrate concentration, and regulates the process based on measurements of both VCD and substrate, may be able to determine appropriate control actions even in the absence of an updated measurement for one of the substrate or the VCD at a particular iteration. This advantage is also present in any control method using a MPC that jointly predicts two controlled variables and regulates the process based on measurements of both of these variables. In such situations, the control of the two variables is more robust to failure of the measurement systems used to measure either one of the two variables, compared to a control method that uses conventional controllers to control the two variables independently. This may be particularly advantageous when one of the measurements is difficult to obtain or obtainable using sensor systems that are prone to failure. For example, measurements for substrates / metabolites may be more difficult to obtain than measurements for viable cell density. As such, coupling regulation of a substrate / metabolite with regulation of VCD as described herein may improve the robustness of the substrate / metabolite regulation process.

**State Observer**

Within the context of the present disclosure, one or more state observers may be used to estimate the value of one or more variables of the bioprocess, such as the values of independent variables of a state space model used by a model predictive controller. A state observer is a model that provides an estimate of one or more internal states of a system (in the present case, the bioprocess), from measurements of inputs and outputs of the system (where the internal states and outputs may overlap, for example a substrate concentration may be both an internal state and an output of the system). In particular, state observers may be used to estimate values for VCD, substrate concentration and even volume. There are many benefits for utilizing state observers. In particular, state observers can be used to provide estimates of states in between at-line or off-line measurements. This allows the control action to be run at a higher frequency than the measurements of states. As another example, state observers can be used to combine multiple measurements of the same parameter. This may be useful when a continuous soft sensor is combined with a more precise infrequent off-line measurement (e.g. lab measurement). For instance, VCD may be estimated via an in-line conductivity or capacitance-based sensor but this measurement has a tendency to drift. VCD may also be measured once a day. A state observer can combine these two measurements in combination with the estimate from the state space model to provide an optimal estimate given the uncertainty in each source. Further, state observers are structured to take into account the relative uncertainty in measurements and the model to optimally handle noisy measurements. As yet another example, state observers can be adapted to handle cases where there is a delay between the time the sample is taken and the result is available. For example, when using a bioreactor with a sensor in the permeate line, rather than in the bioreactor itself, there is a distance of tubing outside of the bioreactor that the permeate needs to pass through before reaching the sensor. This results in a dead time until the permeate from the bioreactor reaches the sensor. When using state observers, this can be considered to update the measurement (e.g. substrate concentration) estimate used by the controller. For example, if there is a known 1 minute of flow time and a known 1 minute measurement time, a 2 minutes delay will be present on any measurement. This time delay can be taken into account in a state observer. For example, the predicted value of a measured variable at a current time k can be obtained based on a more recent measurement (corresponding to k-d, where d is a dead time / time delay) and a state space model that models the change of the measured variable over time. The time delay parameter in such a model may be provided by a user, for example depending on the configuration of the bioreactor system (including the bioreactor and sensor(s) used to provide the measurement, as well as possible physical connections between these),or may be estimated for example by comparing predicted and measured values of the state space variable.

The state observer may be a linear state observer. The state observer may be a continuous time observer. A linear continuous time observer may obtain estimates for a linear system $x = Ax + Bu$ and $y = Cx + Du$ (where x are the true internal states, u are inputs, y are outputs and A, B, C, D are coefficients of the model), as: $\dot{\hat{x}} = A\hat{x} + Bu + L(y - \hat{y})$ and $\hat{y} = C\hat{x} + Du$ where $\hat{x}, \hat{y}$ are the estimates of the internal states and outputs in the observer, L is the observer gain, and the observer error $e = x - \hat{x}$ satisfies the equation $\dot{e} = (A - LC)e$. A state observer may be a Luenberg observer. A Luenberg observer uses a dynamic model of a physical system to estimate its internal states. A Luenberg observer is derived from a linear system assumed to satisfy equations (10a) and (10b):

$$X[k+1] = A*X[k] + B\,U[k] \qquad (10a)$$

$$Y[k] = C*X[k] + D\,U[k] \qquad (10b)$$

where at time k, X[k] are the internal states of the process (e.g. volume, concentration of one or more metabolites, and/or one or more cell densities including viable cell density), U[k] are the inputs of the process (e.g. manipulated variables such as feed, harvest and/or bleed flows), and Y[k] are the outputs of the process (e.g. volume, concentration of one or more metabolites, and/or viable cell density). The Luenberg observer is derived as:

$$\hat{x}(k+1) = A\hat{x}(k) + L[y(k) - \hat{y}(k)] + Bu(k) \qquad (10c)$$

$$\hat{y}(k) = C\hat{x}(k) + Du(k) \qquad (10d)$$

where the variables denoted with a "^" are the variables of the observer, such that y(k) is the predicted output of the state observer and *y(k)* is the measured output of the process.

A state observer may be a Kalman Filter (also known as linear quadratic estimation). A Kalman filter is a method to produce estimates of unknown variables using a dynamic model of a system, known inputs to the system, and measurements of states of the system observed over time, by estimating a joint probability distribution over the variables for each time. The method comprises providing estimate of the current state variables of a system and their uncertainties, and updating these estimates when a new measurement is obtained using a weighted average of the predicted states and the new measurement. The weights are determined to provide more weight to values with lower uncertainty than to values with higher uncertainty, and are calculated from the covariance. The updated estimate is then used to provide a prediction at a next iteration, which is itself updated when a new measurement is obtained. Thus, the method operates iteratively to provide improved estimates of a current state of the system. Kalman filters advantageously deal with uncertainty associated with noise in sensor data, providing better estimates of a system's internal state than can be obtained using sensor measurements alone. For example, when using a Kalman filter to estimate a substrate concentration, a model as described in equations (11), (11a), (11b) may be used. A state observer may be a moving horizon estimation. A moving horizon estimation is a method to produce estimates of unknown variables using a dynamic model of a system, a set of measurements over a window of time (history of past measurements) and an optimization cost function over the estimation horizon. At every iteration (time t) where a new measurement is obtained, the dynamic model is integrated over a time horizon in the past ([t-T,t]) to identify state trajectories that minimize the optimization cost function over the time horizon. The last step of the estimation strategy is then used as an estimate of the current valuable of the variable that was measured. The calculation is then repeated at every iteration, shifting the horizon forward to the new time t. The optimization cost function may be defined as

$$J = \sum_{i=1}^{N} w_m \left( x_{p,i} - x_{m,i} \right)^2 + \sum_{i=1}^{N} w_{\hat{x}} \left( x_{p,i} - \widehat{x_{p,i}} \right)^2 + \sum_{i=1}^{N} w_{p_i} \Delta p_i^{\;2}$$

where $x_{p,i}$ is the i$^{th}$ model predicted variable, $\widehat{x_{p,i}}$ are prior model predictions, $x_{m,i}$ is the ith measured variable, $p_i$ is an estimated parameter related to variable i, $w_m$ is a weighting coefficient reflecting the relative importance of measured values $x_{m,i}$, $w_{\hat{x}}$ is a weighting coefficient reflecting the relative importance of prior model predictions $\widehat{x_{p,i}}$, and $w_{p_i}$ is a weighting coefficient penalizing relative big changes in $p_i$. In the example above where the state observer is used to estimate a substrate concentration, the model provided in equations (9)-(10) may be used as a dynamic model of the system, where $c_A$ is the state variable that is estimated and measured, F is the input to the system, and $r_A$ is the parameter that is estimated. The time horizon may be set in terms of a number of iterations. As explained above, the time horizon may be set to have a length that is sufficiently long to capture complex dynamics that may be expected to occur. This is typically based on experience of the system at hand, and/or using simulations of the system to be controlled. As a safe and conservative selection, a prediction horizon length that includes the time to reach steady state may be used. The choice of a length of the prediction horizon may balance requirements to capture complex dynamics in the system and maintain a practically useful speed of operation of the control loop in view of the computational resources available for calculation. Instead or in addition to this, the length of the prediction horizon may be selected based on the reactor residence time. Methods for calculating an expected residence time or residence time distribution are known in the art. Instead or in addition to this, the length of the prediction horizon may be selected to be able to use the control algorithm as a safety measure, for example in case of a downtime or failure of a sensor. In such a case, the predicted values of one or more manipulated variables over the horizon could be used to control the process while problems with the sensor might be fixed. A moving horizon estimation may be advantageous when there are nonlinearities in the system that is modelled. For example, when the dynamic system that is modelled uses both the VCD and the volume as state variables and the bleed and permeate flow rates as inputs, the system of equations that model the system may not be linear (i.e. they may contain a term that multiplies the two state variables). Any optimization algorithm known in the art may be used to identify a solution that minimizes the cost function of the moving horizon estimation.

When the moving horizon estimation uses a nonlinear dynamic model of the system, the optimization algorithm may be a nonlinear optimizer.

**State Space Model**

A state space model is a model that provides a description of a process to be controlled. A state space model may be used in a state observer to estimate values of parameters that characterise the internal state of the system. A state space model may instead or in addition be used in a model predictive controller, where it is used to generate a prediction of a system's internal state over a prediction horizon. The state space model may be a linear state space model or a linearised state space model. A linearised state space model is a model that comprises linear equations obtained as an approximation of the behaviour of a non-linear model around a stationary point (also referred to as "steady state"). For example, consider a system with internal states x, inputs u and outputs y. When using a state space model to model a substrate concentration, the system's internal states x may include the substrate concentration and consumption rate, the inputs $u$ may include the perfusion rate (feed flow rate), and the outputs may include the substrate concentration. A nonlinear system may be expressed as $\dot{x} = f(x, u)$, $y = g(x, u)$, and a stationary point may be expressed as $\dot{x}_0 = 0$, i.e. $f(x_0, u_0) = 0$. Thus, the state space equations can be expressed in terms of new variables $\Delta x = x - x_0$, $\Delta u = u - u_0$, $\Delta y = y - y_0$ as

$$\dot{\Delta x} = f(x, u) \approx \frac{\partial(f(x_0, u_0))}{\partial x} \Delta x + \frac{\partial(f(x_0, u_0))}{\partial u} \Delta u = A\Delta x + B\Delta u$$

and

$$\Delta u = g(x, u) \approx \frac{\partial(g(x_0, u_0))}{\partial x} \Delta x + \frac{\partial(g(x_0, u_0))}{\partial u} \Delta u = C\Delta x + D\Delta u .$$

The state space model used according to the present disclosure may depend on the controlled variables that are to be controlled using a model predictive controller. For example, when using model predictive control to control a substrate concentration by manipulating the feed flow, a state space model may comprise a single equation expressing the rate of change of the substrate concentration (internal state of the system and output of the system, controlled variable) as a function of the feed flow rate (input, manipulated variable), the concentration of said substrate in said feed (parameter provided by a user or sensor), the volume of the culture (parameter provided by a user or sensor) and the substrate consumption rate (internal state of the system, parameter estimated by solving the system at pseudo steady state). For example, equation (9) may be used:

$$\frac{dc_A}{dt} = \frac{F}{V}\left(c_{A,0} - c_A\right) - r_A \qquad (9)$$

which can also be described in matrix form with equation (10):

$$\dot{x}_{sys} = A \cdot x_{sys,t} + b \qquad (10)$$

where $A = \begin{bmatrix} -\frac{F}{V} & -1 \\ 0 & 0 \end{bmatrix}$ and $b = \begin{bmatrix} \frac{F}{V} \cdot C_{A,0} \\ 0 \end{bmatrix}$ with $\dot{x}_{sys}$ being the rate of change of the substrate concentration and substrate uptake rate in the bioreactor (i.e. $\frac{dc_A}{dt}$ and $\frac{dr_A}{dt} = 0$), $x_{sys,t}$ being the concentration of the substrate in the bioreactor at time t (i.e., $c_A$ ;t=n being the time with most recent data) and the substrate uptake rate at time t (i.e. $r_A$), F being the feed flow rate, $c_{A,0}$ being the substrate concentration in the feed flow rate, being the volume of the bioreactor and $r_A$ being the substrate uptake rate by the cells, assuming that $\frac{dr_A}{dt} = 0$. Assuming further that $\frac{dc_A}{dt} = 0$ (pseudo-steady-state), then it is possible to determine $r_A = \frac{F}{V}\left(c_{A,0} - c_A\right)$ at any one time point, where at a current time point $c_A$ can be measured or estimated, F is known, and V, $c_{A,0}$ are parameters, and at a future time point $c_A$ can be estimated by integration of equation (9) until the future time point, F is known as part of a proposed control strategy, and V, $c_{A,0}$ are parameters. Thus, such a model captures the evolution of substrate concentration as a function of the chosen feed rate, and estimates the current substrate uptake rate by solving the model at pseudo-steady state. As such, the substrate uptake rate need not be known (e.g. empirically determined) prior to conducting the bioprocess, and its value can be adapted during the process so as to reduce departures between the model and the process, thereby further increasing process stability. Note that in equation (9), the cell density is effectively an input of the model, as the substrate uptake rate $r_A$ depends on the cell density (see equation (25) below). The harvest and bleed flows, to the extent that they contribute to loss of substrate, may be ignored as not contributing to changes in concentration of substrate in the reactor, and may not be included in the model by assuming that the volume of the culture is constant (i.e. Ff=Fb+Fh). Alternatively, they may be explicitly included in the model, for example if the volume of the culture cannot be considered to be constant. The substrate concentration (e.g. glucose) may be measured on-line and/or off-line, and this may be used by a state observer (such as e.g. a Kalman Filter) to estimate the current value of the substrate concentration (e.g. taking into account uncertainty, noise and/or possible time delays in the measurements). The measured or estimated substrate concentration may then be used by a model predictive controller to update a feed rate to maintain a set point for the substrate concentration, at an estimated pseudo-steady state of substrate concentration. In such an approach, other controlled variables such as the volume and/or cell concentration may be controlled using one or more further control loops, each using respective controllers which may be e.g. P, PI, PID or model predictive controllers. For example, the volume may be controlled using a control loop that manipulates the permeate (harvest) flow rate. The viable cell density may be controlled using a control loop that manipulates the bleed flow rate.

Note that Equation (9) assumes that the volume of the culture in the bioreactor is not changing, such that Ff = Fb + Fh. Equation (9) can be written in a manner that does not necessarily make this assumption (equation (9a)):

$$\frac{dc_A}{dt} = \frac{F}{V}\left(c_{A,0}\right) - \frac{(F_b+F_h)}{V}c_A - r_A \qquad (9a)$$

As another example, when using model predictive control to control the viable cell density, volume and optionally perfusion rate, a state space model may comprise a kinetic growth model as described further below, such as e.g. equation (11), equations (11) to (14), or simplified versions thereof (see e.g. equation (26) below) expressed in matrix form with equation (10a):

$$X[k+1]=A*X[k]+ B\,U[k] \qquad (10a)$$

where X are the states $x_v$, $x_d$, $x_l$ (respectively the concentrations of live, dead and lysed cells), U are the inputs $F_b$, $F_h$ and $F_f$ (respectively the bleed, permeate and feed flow rates),

$$A = \begin{bmatrix} \mu_{eff} - \mu_d - \frac{F_b}{V} & 0 & 0 \\ \mu_d & -k_l - \frac{F_b}{V} & 0 \\ 0 & k_l & -\frac{F_h+F_b}{V} \end{bmatrix} \qquad B = \begin{bmatrix} -\frac{x_v}{V} & 0 & 0 \\ -\frac{x_d}{V} & 0 & 0 \\ -\frac{x_l}{V} & -\frac{x_l}{V} & 0 \end{bmatrix}$$

A is , B is for sufficiently small sampling time (note, these A and B can be converted for any sampling time using standard discretization formula - and the particular values of the matrices of coefficients A and B depend on the particular equations used for the dynamic model), and the effective growth is a parameter that may be estimated at every iteration of the control method as explained above in relation to the substrate uptake rate by the cells, by solving the model at pseudo steady state. The effective death rate and cell lysing rate may also be parameters that are estimated at every iteration of the control method as explained above in relation to the substrate uptake rate by the cells, by solving the model at pseudo steady state, or they may be maintained at predetermined values (i.e. not automatically updated based on mismatch between model and measurements). Without wishing to be bound by theory, the inventors believe that the effective growth rate is more likely to significantly vary during a bioprocess, and is more directly related to the viable cell density that is controlled by the method, such that it may be more important to automatically update the estimate of the growth rate than that of the death and lysing rate. When using only equation (11) or a similar equation, the effective growth rate may capture contributions of both the growth and death processes, i.e. the effective growth rate may lump together the growth and death rates. Using a more complex predictive model such as one that models dead and lysed cells as well as live cells (e.g. a model that includes equations (11) to (14)) may strengthen the predictive power of the model.

For example, a growth model that explicitly models dead and lysed cells may be able to predict changes to growth kinetics due to changes in media exchange rate. By contrast, a model with a single equation that lumps together growth and death may only respond to changes in growth rate after the growth rate has already been impacted by a change in media exchange rate, through observation of model mismatch (where the state space model is then updated to account for the change in growth rate). Similarly, any change in a variable that is explicitly modelled (or that is directly -e.g. linearly- related to a variable that is explicitly modelled) in the model of equations (11) to (14) will have an impact on the cell related state variables (i.e. live, dead and/or lysed cell concentrations) and this will be captured by the model. By contrast, if a variable is not explicitly modelled in this way then its impact on the cell related state variables may only be captured through update of coefficient that effectively capture their effects (such as an effective growth rate) a *posteriori*. As another example, when using a predictive control to control the viable cell density, volume and a substate concentration, a state space model may comprise a matrix form of a system of equations comprising a kinetic growth model (e.g. equations (11), (11) to (14) or (26)), an equation describing volume change (e.g. equation (27)) and an equation describing the dynamics of the substrate concentration (e.g. equation (25) or (25b)). Thus, a state space model may comprise a kinetic growth model, as described further below. In embodiments, a state space model may comprise a model describing the growth kinetics of cell culture processes as described in WO 2021/165480. The parameters of such a model may be determined as described in WO 2021/165480, for example using a data driven approach such as a partial least square regression model or a machine learning model and/or a Bayesian method as described therein.

The term "kinetic growth model" refers to any model that captures the kinetic of a cell population in a bioprocess. Accordingly, a kinetic growth model may be used to model the number of live cells (and other culture-related parameters) in a bioreactor, for example to make predictions about the number of cells (e.g. viable cell density) in the bioreactor at a future point in time. For example, a kinetic growth model may include one or more differential equations that model the time-dependent behaviour of one or more cell population variables. Cell population variables may be variables that characterise the viable, dead, lysed and/or total cell population in a bioprocess. Typical cell population variables include the viable cell density (VCD), dead cell density, and lysed cell density, which respectively capture the concentration of viable, dead and lysed cells in a bioreactor. In embodiments, a kinetic growth model uses the Monod equation to capture cell growth rates as a function of the concentration of a limiting nutrient. One example of a kinetic growth model is provided in equations (11) to (14) below, which describe respectively changes in the viable cell density $x_v$, the dead cell density $x_d$, the total cell density $x_t$ and the lysed cell density $x_l$ as a function of time:

$$\frac{dx_v}{dt} = \left(\mu_{eff} - \mu_d - \frac{F_b}{V}\right) x_v \qquad (11)$$

$$\frac{dx_d}{dt} = \mu_d x_v - \left(k_l + \frac{F_b}{V}\right) x_d \qquad (12)$$

$$\frac{dx_l}{dt} = k_l x_d - \frac{F_h + F_b}{V} x_l \qquad (13)$$

$$x_t = x_v + x_d + x_l \qquad (14)$$

In equations (11)-(14), $F_b$ and $F_h$ are the bleed and harvest rates, respectively (see above and Figure 1), $V$ is the reactor volume, $\mu_{eff}$, $\mu_d$, and $k_l$ are the effective growth, effective death, and lysing rates, respectively. Equation (11) captures the following assumptions: (i) live cells are formed from live cells at the effective growth rate $\mu_{eff}$; (ii) live cells can exit the reactor through the bleed stream $F_b$ ; (iii) live cells can be transformed into dead cells at the effective death rate $\mu_d$; and (iv) no live cells exit the reactor through the harvest stream $F_h$ (in other words, where a harvest stream is present it includes a perfect cell retention filter - which may be a valid assumption when any cells exiting the bioreactor through the harvest stream represent a negligible amount). The calculation of the effective growth rate is critical to the functioning of this model and is presented in detail below.

Equation (12) captures the following assumptions: (i) dead cells are formed from live cells at the effective death rate $\mu_d$; (ii) dead cells are converted into lysed cells through a first-order process with rate $k_l$ ; (iii) dead cells can exit the reactor through the bleed stream $F_b$ (if present); and (iv) no dead cells exit the reactor through the harvest stream $F_h$. The calculation of the effective death rate is presented in detail below. Equation (13) captures the following assumptions: (i) lysed cells are formed from dead cells at rate $k_l$; (ii) lysed cells can exit the reactor through the bleed stream $F_b$ (if present); and (iv) lysed cells can exit the reactor through the harvest stream $F_h$ (if present). Equation (14) captures the assumption that cells are either viable, dead or lysed. This can be used to calculate one of the variables from the others, for example lysed cells (which typically cannot be measured directly), by closing the balance on living and dead cells.

In embodiments, the death process (captured through the parameter $\mu_d$) can be modelled as resulting from a combination of a base death rate and a toxicity factor, for example as provided in equation (15) below:

$$\mu_d = k_d + k_t \phi_t \qquad (15)$$

In equation (15), $k_d$ is the primary (base) death rate, $k_t$ is the "toxicity rate" and $\phi_t$ is a measure of toxicity. The variable $\phi_t$ may represent the concentration of one or more components that have a toxic effect on cells. In embodiments, $\phi_t$ is assumed to be equal to the concentration of lysed cells $x_l$ (i.e. $\phi_t = x_l$). In other embodiments, $\phi_t$ is assumed to be equal to the concentration of an unknown biomaterial that accumulates in the culture as a result of cell growth and inhibits cells growth and/or is toxic to the cells, which is designated as $\phi_b$ (such that $\phi_t = \phi_b$). The evolution of this variable can be captured for example using equation (16) below:

$$\frac{d\phi_b}{dt} = x_v - \frac{F_h + F_b}{V} \phi_b \qquad (16)$$

which assumes that the unknown product is produced at a rate that is proportional to the viable cell density $x_v$ and that the product can exit the bioreactor through the bleed and harvest streams ($F_b$, $F_h$).

In embodiments, the growth process (captured through the parameter $\mu_{eff}$) is modelled as the product of a growth rate under ideal conditions $\mu_{max}$ (where growth rate is assumed to be maximised), and one or more factors that describe the influence of other system variables on growth. These factors may in some cases take one of three functional forms: substrate limited $\eta_s$, quadratic $\eta_q$ or inhibitory $\eta_i$. The relationship between these factors and the resulting effective growth rate can be captured in equation (17):

$$\mu_{eff} = \mu_{max} \eta_s \eta_q \eta_i \qquad (17)$$

where the correcting factor $\eta_s$ captures the contribution of $N_s$ substrate limiting variables, $\eta_q$ captures the contribution of $N_q$ quadratic influencing variables and $\eta_i$ captures the contribution of $N_i$ inhibitory variables. $N_s$ can be equal to 0 (no substrate limiting variable), 1 (one substrate limiting variable), or any natural number. $N_q$ can be equal to 0 (no quadratic influencing variable), 1 (one quadratic influencing variable), or any natural number. $N_i$ can be equal to 0 (no inhibitory variable), 1 (one inhibitory variable), or any natural number. The correction factors above can be calculated as products of a plurality of correcting factors that each capture the contribution of a substrate limiting / quadratic influencing / inhibitory variable, as shown in equations (18)-(20) below:

$$\eta_s = \prod_{n=1}^{N_s} (\eta_{s,n}) \qquad (18)$$

$$\eta_q = \prod_{n=1}^{N_q} (\eta_{q,n}) \qquad (19)$$

$$\eta_i = \prod_{n=1}^{N_i} (\eta_{i,n}) \qquad (20)$$

In embodiments, inhibitory effects are captured using correction factors of the form provided by equation (21) below:

$$\eta_{i,n} = \frac{1}{\left(\frac{z_n}{\theta_{i,n}}\right)^3 + 1} \qquad (21)$$

where $z_n$ is the concentration of a substance that has a growth inhibitory effect, and $\theta_{i,n}$ is a parameter that represents

the level of $z_n$ above which inhibition occurs. **Figure 3A** shows an example of the value of a growth inhibition factor as a function of the concentration of the growth inhibitory substance whose effect is modelled by the correction factor, for different values of the parameter $\theta_{i,n}$. The variable $\phi_t$ in equations (15) and (16) above may be seen as a special case of an inhibitory effect where the concentration of the inhibitory substance depends on the viable cell density (e.g. because the inhibitory substance is produced by or as a result of the presence of the cells). The variable $\phi_t$ can also sometimes be modelled using a formulation as provided in equation (21). Therefore, equation (17) can in some embodiments be written as:

$$\mu_{eff} = \mu_{max}\eta_s\eta_q\eta_i \frac{1}{\left(\frac{\phi_t}{\theta_t}\right)^3 + 1} \qquad (22)$$

where $\theta_t$ (or $\theta_b$ as the case may be) is a coefficient representing the inhibition in growth from accumulation of the biomaterial $\phi_t$ (where $\phi_t$ can be e.g. equal to $x_l$ or $\phi_b$). As the skilled person understands, in some embodiments there may be more than one substance $\phi_b$, each of which can be modelled using a respective term in equations (17) and (22) and a respective equation (16). Examples of substances that have a growth inhibitory effect and that produced by or due to the presence of the cells in the culture (i.e. substances $\phi_b$) include toxic by-products such as e.g. ammonia. Examples of substances that have a growth inhibitory effect that is not necessarily related to the viable cell density include substances that are included in the culture medium to have a desired effect but that may also have a (ideally slight) growth inhibitory effect on the cell culture (e.g. antibiotics).

In embodiments, substrate limiting effects are captured using correction factors of the form provided by equation (23) below:

$$\eta_{s,n} = \tanh\left(2\frac{z_n}{\theta_{s,n}}\right)^2 \qquad (23)$$

where $\theta_{s,n}$ is a parameter which represents the approximate level below which the variable $z_n$ (substrate that is growth limiting) starts to have a limiting effect on growth. Figure **3B** shows an example of the value of a substrate limitation factor as a function of the concentration of the limiting substrate whose effect is modelled by the correction factor, for different values of the parameter $\theta_{s,n}$. In equation (23) the factor 2 is used to give the parameter $\theta_{s,n}$ an intuitive biological meaning as the approximate concentration $z_n$ below which a limiting effect can be seen (such as e.g. a value such that the inhibitory function crosses the -0.95 threshold when the value of $z_n$ falls below that of $\theta_{s,n}$). Any other value can be used for this factor, resulting in the same behavior with the effect that the parameter $\theta_{s,n}$ is adjusted accordingly and no longer has the same intuitive interpretation. In particular, this factor can be omitted entirely (i.e. set to 1). Similarly, in equations (21) and (22), the term that is cubed (i.e. ($z_n$ /$\theta_{i,n}$)) may comprise a coefficient that gives the parameter $\theta_{i,n}$ an intuitive biological meaning as the approximate concentration $z_n$ above which a limiting effect can be seen (such as e.g. a value such that the inhibitory function crosses the -0.95 threshold when the value of $z_n$ reaches that of $\theta_{i,n}$, for example 0.37). Examples of substances that have a substrate limiting effect include nutrients such as glucose, amino acids, etc.

In embodiments, quadratic effects are captured using correction factors of the form provided by equation (24) below:

$$\eta_{q,n} = \exp\left[-\frac{1}{25}\left(\frac{z_n - \mu_{q,n}}{\theta_{q,n}}\right)^2\right] \qquad (24)$$

where $\mu_{q,n}$ is a parameter that represents the target value (i.e. the value at which maximum growth occurs) and $\theta_{q,n}$ is a parameter that represents the "spread"' of the effect. The factor 1/25 used to give the parameter $\theta_{q,n}$ an intuitive meaning where a value of $\theta_{q,n}$ = 1 means that the quadratic effect crosses a 95% threshold at $\pm 1$ around the target value $\mu_{q,n}$. Any other value can be used for this factor, resulting in the same behavior with the effect that the parameter $\theta_{q,n}$ is adjusted accordingly and no longer has the same intuitive interpretation. In particular, this factor can be omitted entirely (i.e. set to 1).The use of factors that provide an intuitive interpretation of parameters such as $\theta_{s,n}$ and $\theta_{q,n}$ may be advantageous in that it may make it easier for a user to set these parameters (using e.g. biological knowledge or assumptions) or realistic boundaries for these parameters. **Figure 4A** shows an example of the value of a quadratic effect factor as a function of the concentration of the substance whose effect is modelled by the correction factor, for different values of the parameter $\mu_{q,n}$ with a fixed value of $\theta_{q,n}$ =1. **Figure 4B** shows an example of the value of a quadratic effect factor as a function of the concentration of the substance whose effect is modelled by the correction factor, for different values of the parameter $\theta_{q,n}$ with a fixed value of $\mu_{q,n}=5$.

As the skilled person understands, other equations can be used instead or in addition to the above to model cell population variables and factors that affect these variables. These equations together may form a state space model in that they describe the evolution of the state of the cell culture (also referred to herein as "cell state") in terms of a series of state variables $x_v$, $x_d$, $x_t$, $x_l$ as a function of inputs to the system including e.g. the concentration of various substances that influence the cell state.

Thus, a state space model may comprise a matrix form of a system of equations that comprise a kinetic growth model and/or one or more equations that capture the evolution of the concentration of one or more metabolites in the bulk culture (including in particular one or more nutrients/substrates, by-products and/or the desired product), as described below (such a e.g. equation (25)). Note that the model provided in equations (9) (or 9(a)) and (10) is an example of a model that includes a single equation that captures the evolution of the concentration of a single metabolite (in this case, a substrate) in the bulk culture. The flows of metabolite may be expressed as mass flows or molar flows (as the latter can be converted to the former and vice-versa using molar mass, such that the conservation of mass expressed in equation (25) is verified regardless of the units chosen), and the skilled person would be able to convert one into the other. As such, references to mass flows are intended to encompass the use of the corresponding molar flows with corresponding adjustments for consistency of units within an equation. Similarly, references to concentrations may refer to the mass or molar concentrations. The flow of a metabolite into the bioreactor depends on the value of the feed flow $F_F$ and the concentration of the metabolite in this flow. The flow of the metabolite out of the bioreactor depends on the value of the permeate flow $F_H$ and the value of the bleed flow $F_B$, and the concentration of the metabolite in these respective flows. The consumption and secretion of a metabolite by the cells in the bioreactor depends on the viable cell density in the reactor and on a variable called the "specific transport rate" (sometimes also referred to as "metabolic rate"), which can also be referred to as ""specific consumption rate" (typically, if it is negative, i.e. the metabolite is being consumed by the cells) or the "specific secretion/production rate" (typically, if it is positive, i.e. the metabolite is being produced by the cells) of the metabolite by the cells. Therefore, the material balance for a metabolite i can be written for a general system (e.g. as illustrated on Figure 1), as equation (25) below:

$$\frac{d(m_i)}{dt} \quad = \quad \frac{F_F}{V} * m_{F,i} \quad - \quad \frac{F_H}{V} * m_{H,i} \quad - \quad \frac{F_B}{V} * m_{B,i} \quad + \quad \delta_{m,i} * x_v \quad (25)$$

which expresses the conservation of mass in the system at any time point t. In equation (25), $\delta_{m,i}$ is the specific transport rate of metabolite i by the cells in the culture, $m_i$ is the concentration of metabolite i in the reactor, V is the volume of the culture in the bioreactor, $m_{F,i}$ is the concentration of metabolite i in the feed flow, $m_{H,i}$ is the concentration of metabolite i in the permeate flow, $m_{B,i}$ is the concentration of metabolite i in the bleed flow, $x_v$ is the viable cell density in the reactor, and $F_F$, $F_H$ and $F_B$ are the volumetric feed, harvest and bleed flow rates, respectively (although mass flow rates can equally be used with the appropriate coefficients for densities of the respective flows). In embodiments, the term $\delta_{m,i} *$ $x_v$ can be replaced by a term $\delta_{m,i} * x_v * \left(\frac{m_i}{m_i+\varepsilon}\right)$ where $\varepsilon$ is a constant that is chosen to ensure that the values of $m_i$ that are below the detection limit for the metabolite do not cause errors in the estimation of specific transport rates. Typically, $\varepsilon$ is chosen to be approximately equal to the detection limit for the metabolite (e.g. it can be chosen as 0.05 where the metabolite concentration is standardised). Equation (25) assumes that the permeate flow 28A contains the only material leaving the system through the auxiliary harvest flow 28C (i.e. the auxiliary harvest flow and the return flow do not need to be included in the model as the metabolite only leaves the system through the permeate flow), and that the cell retention device 28 functions such that the permeate flow 28A can be assumed to contain no cells. Equation (25) can be adapted to include an auxiliary harvest flow 28C (and corresponding $m_{A,i}$ and density $\rho_A$ if a mass flow rate is used) and a return flow 28B (and corresponding $m_{R,i}$ and $\rho_R$). Further, equation (25) can be amended to model the removal of some cells through the permeate flow. In other words, additional terms can be added to equation (25) and some can be removed depending on the set-up of the bioprocess and the assumptions made. Equation (25) (referred to herein as "material balance model" or "bulk metabolite concentration model") captures the relationships between extracellular fluxes of metabolites and the concentration of the metabolites in the culture medium. The model does model intracellular metabolic fluxes, which may be captured using metabolic models that model fluxes along reactions that together form the cell's metabolic network, and which are significantly more complex (and thus more difficult to solve and requiring extensive data and/or assumptions to parameterise). As such the models used herein advantageously provide information about the metabolic state of the cells while remaining relatively simple to solve and parameterised, allowing to obtain important system state variables such as the specific transport rates of metabolites (as will be explained further below) in a simple and efficient manner.

Further, equation (25) can be simplified by making a few assumptions. For example, assuming that the metabolite concentration is the same everywhere in the culture medium in the bioreactor, and hence also in the permeate flow and in the bleed flow (in other words, assuming that concentration gradients within the reactor are negligible such that $m_{B,i} = m_{H,i} = m_i$), and that the number of cells lost in the bleed and permeate flows is negligible, equation (25) can be written as:

$$\frac{d\,(m_i)}{dt} \quad = \quad \frac{F_F}{V} * m_{F,i} \quad - \quad \frac{(F_H + F_B)}{V} * m_i \quad + \quad \delta_{m,i} * (x_v) \quad (25a)$$

Equation (25a) corresponds to equation (9a), using the formalism of equation (25). Assuming further that the volume of the culture is constant (i.e. $F_F = F_H + F_B$), that the flows are constant, and using a first-order finite difference approximation for the derivative, equation (25a) can be resolved for the metabolite specific consumption/secretion rate at time $t_k$ as:

$$\delta_{m,i}(t_k) = \frac{V*(m_{i,k+1} - m_{i,k}) - F_F * m_{F,i,k}*(t_{k+1} - t_k) + F_F * m_{i,k}*(t_{k+1} - t_k)}{V * IVCD_k} \quad (26a).$$

Note that equation (9) is also a simplification of equation (25) which considers that the volume of the culture is constant, such that Ff=Fb+Fh. Thus, assuming that the volume of the culture is constant, equation (25a) can be re-written as equation (25b), which corresponds to equation (9):

$$\frac{d\,(m_i)}{dt} \quad = \quad \frac{F_F}{V} * (m_{F,i} \quad - \quad m_i) + \quad \delta_{m,i} * (x_v) \quad (25b)$$

where in equation (9) the viable cell density and cell specific uptake rate are combined in a single coefficient $r_A$ that is estimated. When the viable cell density is instead explicitly modelled, for example using a kinetic growth model such as equation (11), it may be advantageous to use equation (25b) instead of equation (9). For example, a state space model may include an equation capturing the change in concentration of a metabolite (e.g. a substrate), such as equation (25), (25a) or (25b), and one or more equations capturing the change in cell density, such as equation (11) or equations (11) to (14). In such embodiments, the substrate concentration and viable cell density may be measured at every iteration and this may be used by a state observer to obtain estimates of the substrate concentration and viable cell density as well as estimates of the cell specific uptake rate and effective growth rate by integration of the state space model. These estimates may be used by a model predictive controller to identify a control strategy to jointly control the viable cell density and substrate concentration.

Any methods to calculate an integrated viable cell density may be used in the methods described herein. For example, $IVCD_k$ may be calculated using equation (27):

$$IVCD_k = \left(\alpha\, x_{v,k} + \beta\, x_{v,k+1}\right) * (t_{k+1} - t_k) \quad (27)$$

where the coefficients $\alpha$ and $\beta$ weight the relative influence of the two viable cell density values and are such that $\alpha + \beta = 1$. For example, the two values may be weighted identically, i.e. $\alpha = \beta = 0.5$. In embodiments, $\alpha$ and $\beta$ are chosen such that $\alpha > \beta$ (for example a=0.6 and $\beta$=0.4). These coefficients may be chosen to reflect the observed cell growth behaviour, and may be chosen independently for each time point. Where exponential growth can be assumed, the integrated viable cell density can be calculated using a log transform. For example, the integral cell density may be calculated using equation (27a):

$$IVCD_k = x_{v,k} * e^{u_k(t_{k+1} - t_k)} \quad (27a)$$

where

$$\mu_k = \frac{ln\left(x_{v,k+1}/x_{v,k}\right)}{t_{k+1} - t_k} \quad (27b)$$

The equations for $\delta_{m,i}$ at time k as described above can be solved using the known (typically measured) biomass and metabolite concentrations at times k and k+1 to obtain a metabolite transport rate at every time point value where the above measurements are available. Further, this can be performed individually for each measured metabolite. The resulting metabolite transport rates are expressed as an amount of metabolite (mass or moles) per cell per unit of maturity (i.e. typically per unit of time). Note that in equations for specific transport rates, the signs of all the terms can be inverted depending on whether a negative rate is taken to mean that the cells consume the metabolite and a positive rate is taken to mean that the cells produce the metabolite (i.e. the rate is seen from the perspective of the culture medium), or the opposite (i.e. a positive rate means that the cells consume the metabolite and a negative rate means that the cells produce the metabolite, in other words the rate is seen from the perspective of the cells compartment).

A state space model may be a self-adapting state space model. A self-adapting state space model refers to a state space model where one or more coefficients in the model are updated during operation of the bioprocess based on one or more measurements of state space variables collected during operation of the process. This is by contrast to e.g. simply using a constant value for these coefficients throughout operation of the bioprocess, or even using a different value for these coefficients as provided by a user for different iterations of the control loop. The use of automatically updated coefficients in the state space model further improves the stability and reliability of the model predictive control as the model more accurately reflects the current state of the bioprocess. This is particularly important in the context of a perfusion bioprocess where cells may be maintained in culture during prolonged periods of time, as the metabolism of the cells themselves may vary in that time (resulting in e.g. different growth rates, different substrate consumption rates, etc.), as well as relevant characteristics of the bioreactor system (such as e.g. performance of the filtration system for the permeate). For example, the state space model may include kinetic coefficients representing a specific growth rate and/or a specific substrate consumption rate. The control method may compare the predicted value of the VCD and/or substrate concentration to measurements collected from the process. Deviations between the predicted and measured values may be used to generate optimal updates to predicted values given measurement noise and model uncertainty, for example using a state observer (e.g. a Kalman Filter, as described above). Instead or in addition to this, deviations between the predicted and measured values may be used to update kinetic coefficients in the state space model to continuously improve predictions of future process states. The term "process states" may refer to the states defined as states in the states space model. Thus, the state space models described above may be updated at regular intervals, such as e.g. every iteration, every x iterations, or every iteration of the control loop that satisfies one or more criteria (e.g. every iteration that satisfies the criterion of having a more accurate (compared to measurements obtained at all iterations), measurement for one or more variables of the state space model, such as e.g. an off-line measurement). Updating the state space model may comprise updating the value of one or more coefficients of the model, such as e.g. one or more kinetic coefficients. This may be achieved by considering the coefficients of the model to form part of the state space variables to be estimated at each iteration where the model is to be updated. In other words, instead of being fixed to predetermined values provided by a user, one or more coefficients of the state space model may be considered to represent state space variables that are estimated by the state observer. The update values for these coefficients are then used by the model predictive controller to predict the trajectory of the process in the prediction horizon, and hence determine an optimal control strategy. For example, in the model of equations (9) and (10)), the value of $r_A$ may be updated based on a comparison between a predicted and measured value of the substrate concentration $c_A$. For example, the process may proceed as follows, with k referring to iterations of the control method (e.g. when a new on-line glucose measurement is received). At k=1, the initial measurements and an initial (user defined) value of $r_A$ are used. This is used to integrate equation (9) to predict the value of $c_A$ and $r_A$ in between measurements of the substrate. At k=2, a Kalman filter is used to calculate new (most recent) estimates of the system state, i.e. $c_A$ and $r_A$. These are used to integrate equation (9) for the upcoming interval. This is repeated for a number of iterations, such as e.g. approx. 5 iterations, in order to obtain a number of estimates of the system states $c_A$ and $r_A$ for optimal performance of the Kalman filter. At k=i (typically a number of iterations set by the user), the model predictive controller is activated to determine a first control action for the manipulated variable (e.g. feed flow rate). At k=i+1, new Kalman Filter estimates of the system states are obtained. These are used as a basis to integrate equation (9) at the beginning of the next interval to determine the control action to be taken for the manipulated variable via the model predictive controller, by integrating the ODE model at a certain frequency until a next on-line measurement is received at k=i+2 . As another example, in the model of equations (25) /(25a), the value of $\delta_{m,i}$ may be updated based on a comparison between a predicted and measured value of the metabolite concentration $m_i$. As another example, in the model of equations (11) to (14), the value of one or more of the effective growth, effective death, and lysing rates ($\mu_{eff}$, $\mu_d$, and $k_l$), may be updated based on a comparison between a predicted and measured value of the viable cell density $x_v$, lysed cell density $x_l$ and dead cell density $x_d$, if available. In embodiments, in the model of equations (11) to (14), the value of the effective growth rate ($\mu_{eff}$) may be updated based on a comparison between a predicted and measured value of the viable cell density $x_v$. In such embodiments, the dead and lysed cell densities may be updated based on a comparison between a predicted and measured value of the viable cell density $x_v$ using equation (14) and a state observer.

A simplified version of equations (11) to (14) combines all effects of growth and death rate into a single variable,

$\mu_{tot}$ (equation (26)). In this form, and allowing for volume changes, we can write a new set of equations including equation (26) and equations (27)-(28):

$$\frac{dx_v}{dt} = \left(\mu_{tot} + \frac{F_h - F_f}{V}\right) x_v \qquad (26)$$

$$\frac{dV}{dt} = F_f - F_h - F_b \qquad (27)$$

$$\frac{d\mu_{tot}}{dt} = 0 \qquad (28)$$

Note that equation (26) can also be used with the assumption that no volume change is expected, i.e. $F_f = F_b + F_h$. However, allowing for the volume to change by including equation (27) or an equivalent thereof that models volume change enables volume control to be performed jointly with control of other variables, such as the viable cell density in the example using equations (26)-(28). Equation (27) can also be used together with a kinetic growth model that does not make the simplifications in equation (26) (e.g. equation (11) or equations (11)-(14)), and/or together with a model that captures the rate of change of concentration of one or more metabolites (e.g. equations (25) or (25a)). This enables respectively the joint control of the volume as well as the cell density (e.g. where equation (27) is combined with equation (11) or equations (11)-(14)) and concentration of one or more metabolites (e.g. where equation (27) is combined with equations (25) or (25a)), or all of the above (where equation (27) is combined with (i) equation (11) or equations (11)-(14), and (ii) equations (25) or (25a)).

With any of these choices of systems of equations, linearizing around a steady state value determined for a given perfusion rate, viable cell density, and volume, the system can be approximated in canonical linear form as:

$$\frac{dx}{dt} = Ax + Bu \qquad (10a)$$

$$y = Cx \qquad (10b')$$

where when using equations (26)-(28), A, B and C are given by:

$$A = \begin{bmatrix} \mu_{tot} + \dfrac{F_h - F_f}{V} & -x_v \dfrac{F_h - F_f}{V^2} & x_v \\ 0 & 0 & 0 \\ 0 & 0 & 0 \end{bmatrix}$$

$$B = \begin{bmatrix} -\dfrac{x_v}{V} & \dfrac{x_v}{V} & 0 \\ 1 & -1 & -1 \\ 0 & 0 & 0 \end{bmatrix}$$

$$C = \begin{bmatrix} 1 & 0 & 0 \\ 0 & 1 & 0 \end{bmatrix}$$

After discretization (see equation (10)), equations (10a) and (10b) can be used in a standard Luenberger observer:

$$\hat{x}(k + 1) = A\hat{x}(k) + L[y(k) - \hat{y}(k)] + Bu(k) \qquad (10c)$$

$$\hat{y}(k) = C\hat{x}(k) \qquad\qquad (10d')$$

Note that the poles of the observer (A-LC) can be adjusted by adjusting L to provide an emphasis on updating the estimated coefficient(s) (in the example above, the overall growth/death rate) while favoring (to a user-defined extent) measured values of the process states (in the example above, the viable cell density and volume).

Updating the sate space model may also be referred to as "parameterising" or "reparameterising" the state space model. Thus, the method of any aspect may comprise parameterising the state space model. Parameterising the state space model may comprise obtaining a measurement corresponding to a state variable for a bioprocess, predicting the value of one or more state variables for the bioprocess using the state space model with a first set of parameters, and determining whether the measurement is within a range of the values predicted by the state space model with the first set of parameters. Parameters of the state space model may include coefficients associated with the state space model, such as e.g. any coefficient of equations (9) to (25)/(25a). The method may further comprise providing a second set of parameters based on the difference between the measurement and the values predicted by the state space model with the first set of parameters.

**Systems**

**Figure 5** shows an embodiment of a system for controlling a bioprocess according to embodiments of the present disclosure. The system comprises a computing device 1, which comprises a processor 101 and computer readable memory 102. In the embodiment shown, the computing device 1 also comprises a user interface 103, which is illustrated as a screen but may include any other means of conveying information to a user such as e.g. through audible or visual signals. The computing device 1 is operably connected, such as e.g. through a network 6, to a bioprocess control system comprising a bioreactor 2 (such as e.g. as illustrated on Figure 1), one or more sensors 3, and one or more effectors 4. The computing device 1 may be a smartphone, tablet, personal computer or other computing device. The computing device 1 is configured to implement a method for controlling a bioprocess, as described herein. In alternative embodiments, the computing device 1 is configured to implement part of a method for controlling a bioprocess, and to communicate with a remote computing device (not shown), to implement other parts of a method of controlling a bioprocess, as described herein. For example, the computing device 1 may be a distributed control unit and may communicate with a remote computing device that implements the functions of one or both of a state observer and model predictive controller. Communication between the computing device 1 and the remote computing device may be through a wired or wireless connection, and may occur over a local or public network such as e.g. over the public internet. Each of the sensor(s) 3 and optional effector(s) 4 may be in wired connection with the computing device 1 (or the remote computing device, if present), or may be able to communicate through a wireless connection (i.e. through a network 6), such as e.g. through WiFi, as illustrated. The connection between the computing device 1 and the effector(s) 4 and sensor(s) 3 may be direct or indirect (such as e.g. through a remote computer). The one or more sensors 3 are configured to acquire data that relates to a bioprocess being performed in the bioreactor 2). The one or more effectors 4 are configured to control one or more manipulated variables of the bioprocess being performed in the bioreactor 2.

The one or more sensors 3 may each be on-line sensors (sometimes also referred to as "inline sensors"), which automatically measure a property of the bioprocess as it progresses (with or without requiring a sample of the culture to be extracted), or off-line sensors (for which a sample is obtained whether manually or automatically, and subsequently processed to obtain the measurement). Each measurement from a sensor (or quantity derived from such a measurement) represents a data point, which is associated with a time (or corresponding maturity) value. The one or more sensors 3 may comprise a sensor that is configured to record the biomass in the bioreactor 2, referred to herein as a "biomass sensor". This is typically in the form of a viable cell density or parameter from which viable cell density can be estimated. The biomass sensor may record a physical parameter from which the biomass in the bioreactor (typically in the form of the total cell density or the viable cell density) can be estimated. For example, biomass sensors based on optical density or capacitance are known in the art. The one or more sensors may further comprise one or more sensors that measure the concentration of one or more metabolites (where the term "metabolites" as used herein includes both substrates of cellular metabolism such as e.g. glucose, as well as products of the metabolism of the cells in the bioreactor), referred to herein as "metabolite sensors". A metabolite sensor may measure the concentration of a single or a plurality of metabolites (such as e.g. from a few metabolites to hundreds or even thousands of metabolites), in the culture as a whole, the culture medium compartment, the biomass compartment (i.e. the cells as a whole), or specific cellular compartments. Examples of metabolite sensors are known in the art and include NMR spectrometers, mass spectrometers, enzyme-based sensors (sometimes referred to as "biosensors", e.g. for the monitoring of glucose, lactate, etc.), etc. Most of the commonly used metabolite sensors measure the concentration of a metabolite in the culture medium. Thus, unless specified otherwise, references to the concentration of a metabolite refer to the concentration of the metabolite in the culture medium in the bioreactor.

As used herein, sensors 3 (such as e.g. metabolite and biomass sensors) may also refer to systems that estimate the concentration of a metabolite or the amount of biomass from one or more measured variables (e.g. provided by other sensors). For example, a metabolite sensor may in practice be implemented as a processor (e.g. processor 101) receiving information from one or more sensors (e.g. measuring physical/chemical properties of the system) and using one or more mathematical models to estimate the concentration of a metabolite from this information. For example, a metabolite sensor may be implemented as a processor receiving spectra from a near infrared spectrometer and estimating the concentration of metabolites from these spectra. Such sensors may be referred to as "soft sensors" (by reference to their "measurements" being obtained using a software rather than by direct measurement). The one or more sensors 3 may further include one or more sensors that measure further process conditions such as pH, volume of the culture, volumetric / mass flow rate of material in / out of the bioreactor, medium density, temperature, etc. Such sensors are known in the art. Whether one or more sensors 3 that measure further process conditions are necessary or advantageous may depend on at least the operating mode and the models used in the state observer and/or controller. For example, it may be advantageous to include one or more sensors measuring the amount and/or composition of the flows entering and/or leaving the bioreactor. Further, the one or more sensors may include a sensor that measures the volume of liquid in the bioreactor (such as e.g. a level sensor or weighing scales). The measurements from the sensors 3 are communicated to the computing device 1, which may store the data permanently or temporarily in memory 102. The computing device memory 102 may store one or more state observer models and/or one or more controller models as described herein. The processor 101 may execute instructions to provide targets for manipulated variables (the feed, harvest and bleed flow rates) using these models and the data from the one or more sensors 3 and/or simulated data, as described herein (such as e.g. by reference to Figures 2, 3 and 4).

The control methods and systems described herein have many benefits over the prior art. For example, they enable improved continuous processing by stabilizing flow streams. Stabilizing flow streams, and especially the permeate flow is important for enabling integrated upstream/downstream continuous operation. The harvest stream may be connected to downstream processes such as purification processes, which may be dependent on a consistent permeate flow rate. Further, the methods and systems described herein may improve process efficiency and quality, by ensuring that cells are provided sufficient nutrients through control of a substrate such as glucose. This strategy can also be used to prevent overfeeding leading to wasted media or even overflow metabolism in the presence of excess nutrients. Controlling to a specific glucose concentration has been shown to have a positive influence on metabolism including influencing product quality. The present methods and systems additionally minimize process perturbations, by minimizing adjustments in flow streams. This steadies the process, resulting in improved cell performance as cells generally perform better in steady environments. This also has beneficial stabilizing qualities for controller other critical process parameters such as dissolved oxygen (DO), pH. Achieving stable perfusion operation in perfusion mode is an important and unmet challenge in the field.

### *Examples*

Exemplary methods of controlling a perfusion flow bioprocess will now be described.

### *Example 1*

#### *Introduction*

Continuous bioprocesses are getting ever more popular in the industry. For example, in upstream bioprocessing (i.e. all parts that precede the fermentation step in which cells are used e.g. to produce a desired product), perfusion processes are commonly employed to reach high viable cell concentrations (VCC; $>30\cdot10^6$ c·mL$^{-1}$). These can then be maintained over an extended period of time (>30 days) in such perfusion processes for the productive phase. The precise and robust control of substrate concentration(s) is a major aim in such processes as it helps to ensure stable and reliable process performance in terms of cell growth and productivity. Therefore, at least the feed flow rate needs to be controlled, since this determines how much cell culture media is provided per unit of time. Currently, there are different approaches of varying technical complexity and differing accuracy to control the feed flow rate.

The simplest way to control the feed flow rate is to define a ratio of feed flow rate and VCC that is to be maintained. For example, a flow rate may be set at 1 litre/day for a VCC of $20\cdot10^6$ cells/ml. The ratio of these two quantities may be kept constant as the cells grow (increasing the VCC). This is commonly referred to as "cell specific perfusion rate" (CSPR) control, as discussed above. In addition to the drawbacks already mentioned above, this approach requires reliable VCC data, and its performance is dependent on the frequency of acquisition of such reliable VCC data.

A slightly more complex approach combines the use of an on-line biomass sensor and an on-line sensor for substrate(s), e.g., glucose. This enables independent control of VCC and glucose concentration, where the feed flow rate can then be controlled according to a defined substrate concentration setpoint by using a standard control algorithm (e.g. PI or PID). This approach still has drawbacks at least in that they are not able to consider changes in the cell size, i.e., viable cell volume, if conducted in single-frequency mode and if on-line values are not referenced against off-line measurements. Indeed, on-line measurements of VCC typically use capacitance sensors with a single frequency, which measure a signal indicative of viable cell volume rather than cell number. Note that this is also the case when these measurements are used in CSPR control as described above. Solutions that attempt to correct for this by working with multi-frequency scanning capacitance sensors have been proposed, but these still require appropriate empirical data and respective data analysis for each bioprocess to be controlled. Relatively few approaches have been described for the joint control of substrate and biomass. For example, Abbate et al. (Experimental Validation of a Cascade Control Strategy for Continuously Perfused Animal Cell Cultures. Processes 2020, 8, 413) describe a cascade control structure to regulate biomass and glucose concentrations, including an inner loop that implements a feedback linearisation using on-line estimates of the biomass growth rate and glucose uptake rate, and an outer loop using two PI controllers (one for the biomass inner loop input and one for the glucose inner loop input) to control the linearized process. The approach only controls the feed rate, using the outputs from the PI controllers. No other variable can be controlled through this model. This is a relatively complex approach due to its underlying model and dependent control loops, which means that the approach is not easily transferable to a different bioreactor system, Instead, it needs to be redesigned and tuned for every system. Further, this approach does not consider potential measurement noise and delay of the substrate sensor, which could impact the reliability and stability of the controlled process. Additionally, the approach is unable to include restrictions on possible step changes and step change width of the feed flow rate. Such step changes may also be detrimental to the stability and reliability of the controlled process. Finally, this approach is also unable to account for potential physiological and metabolic changes in long-term cultivations, and requires reliable and frequent VCC data.

The inventors therefore set out to provide a more sophisticated approach that satisfies operator's needs with respect to process efficiency, reliability and robustness and that can be implemented with reasonable effort independent of the bioreactor and the biological system.

*General approach to control of a perfusion bioreactor*

This example describes an approach for operating a perfusion bioreactor using independent control loops for: (i) the working volume / level, (ii) the VCC, and (iii) the substrate concentration. The volume is controlled based on the measured bioreactor weight via control of the permeate using PID control. The VCC is controlled based on on-line biomass measurements via control of the bleed flow using PID control. The substrate concentration is controlled based on on-line measurements of the substrate via control of the feed flow using an advanced perfusion control (APC) algorithm.

The bioreactor used in this example is a bioreactor with a cell retention device, connected to a control tower that controls process parameters (e. g. pH, DO, Temp. and agitation) and individual pumps for the control of the permeate, bleed and feed flow rates. The bioreactor is further equipped with on-line probes to measure the VCC (to control the bleed flow rate to maintain a defined VCC setpoint, using PID control) and to measure a substrate, e. g. C- or N-sources (to control the feed flow rate using an APC algorithm to maintain a defined concentration setpoint of this substrate in the bioreactor).

*Advanced Predictive Control of feed flow rate*

Before starting the APC algorithm and thus feed flow control at the beginning/start of a perfusion cultivation, the following parameters are provided to the algorithm:

- a mathematical model describing the cellular utilization of the substrate (state space model),
- a concentration setpoint for this substrate that is relevant to the process performance,
- the concentration of the substrate in the fresh perfusion medium supplied,
- an initial substrate uptake rate,
- the working volume of the cultivation,
- the measuring interval of the on-line probe to measure the substrate,
- an initially constant feed flow rate,
- minimum and maximum possible feed flow rates (i.e. low and high thresholds for the feed flow rate),
- minimum and maximum increment of the feed flow rate (i.e. maximum desired step for the feed flow rate).

In a first stage, the perfusion is started using the initial constant feed flow rate provided, and the substrate is measured continuously during the process in a certain and defined time interval with the substrate on-line probe .This initial period is used to collect enough measurements to obtain good predictions from the Kalman Filter. Typically, only a few measurements may be sufficient to have good Kalman filter estimates, such as e.g. 5 or 6 iterations (measurements).

After this initial starting phase, the control algorithm is activated. With each new measurement of the substrate an estimate of the measurement is calculated to consider potential measuring errors, using a Kalman filter as further described below. The estimate of the measured concentration is used to calculate changes on the feed flow rate using a predictive model required to maintain the previously defined concentration setpoint of the substrate for the upcoming measuring interval (see Model Predictive Control below). The feed flow rate is then adapted according to the predicted change required to maintain the previously defined concentration setpoint for the substrate for the next measuring interval. The predictive model is used to simulate the course of the concentration of the substrate until the next measurement of the substrate is obtained. This is used by the model predictive controller to identify a control strategy that is optimal over a prediction horizon.

The described approach including the APC algorithm for feed flow control addresses current obstacles regarding efficient and precise perfusion control by (i) using completely independent control loops for the three flow rates permeate, bleed and feed, (ii) providing a control method that is applicable to many different bioreactor systems and cell types, i.e., plug-and-play solution with minimal a-priori knowledge required for rapid practical feasibility, (iii) compensating for measurement errors (e.g. random noise) as well as accounting for measuring dead times of the on-line substrate probe (within the APC, used as input for the APC to perform feed control), (iv) enabling prediction of changes of the feed rate required for multiple upcoming intervals to maintain respective target concentrations (i.e. increase flexibility in the setting of target substrate concentration), and (v) providing a control solution that is robust to potential technical short-term failures that deflect individual measurements, since required feed flow rates for upcoming intervals were already predicted. Additionally, the control approach ensures optimal media consumption as feed is controlled based on substrate concentration, ensuring that there is no over- or under-feeding. Finally, the behaviour of the system in terms of simulated substrate concentration and uptake rate in between two measurements can be displayed to a user e.g. for monitoring purposes.

*Proof of concept: APC for glucose control*

**Figure 6** illustrates the set-up of this proof of concept experiment. This was performed in a Biostat® RM perfusion bioreactor 700. The following parameters were controlled with individual control loops: level, VCC and glucose as substrate.

The level (volume) was controlled by adjustment of the permeate flow 610. The internal perfusion membrane of the Flexsafe® RM perfusion bag is used as cell retention device. Filtrate 610 is removed from the bioreactor to maintain a constant working volume using a gravimetrical PID flow controller 630 that uses the bioreactor weight of the Biostat® RM (from load cells 640) as input - collected via a distributed control unit 650 (control tower).

The VCC was controlled by adjustment of the bleed flow 660. The biomass was measured on-line using a biomass sensor 660 (BioPAT® ViaMass from Sartorius). This is used to keep the VCC constant (via Bleed F2 660) using a PID controller 670.

The glucose concentration was controlled by adjustment of the feed flow 680. Within the permeate 610, a glucose sensor 690 (in particular, the bypass module of BioPAT® Trace from Sartorius, a system for monitoring of glucose and lactate) is installed to measure the glucose concentration on-line. This is transferred to a computing device 620, where this measurement signal is transferred via OPC interconnections from the BioPAT® Trace software to the MFCS/win 3.1 to MATLAB, which is deployed to run the advanced perfusion control (APC) algorithm for control of the glucose concentration. The APC algorithm consists of three subunits: 1) ordinary differential equation (ODE) model, 2) Kalman filter and 3) model predictive control (MPC), described in detail below. At the beginning of the APC algorithm, the variables and parameters listed in Table 1 below are created and defined to initialize the algorithm.

**Table 1.** Variables that are defined for initialisation of the glucose control APC. Variables subscripted with 't' are updated with every new iteration, i. e. once a new on-line measurement is conducted every interval n (h=hours).

| Variable | Symbol | Formula | Unit | Remark |
|---|---|---|---|---|
| Working volume | $V$ | constant | L | - |
| Perfusion rate | $F_t$ | - | mL/h | Rate of supply of fresh perfusion media |
| Substrate concentration | $C_{A,0}$ | constant | g/L | Substrate concentration in the feed |
| Substrate concentration | $C_{A,inf}$ | constant | g/L | Setpoint substrate concentration |
| Substrate concentration | $C_{A,mess,t}$ | - | g/L | Most recent substrate measurement |

(continued)

| Variable | Symbol | Formula | Unit | Remark |
|---|---|---|---|---|
| Substrate uptake rate | $r_{A,t}$ | Initial value required | g/(Lh) | Total consumption rate of nutrient |
| Measuring interval | $t_k$ | constant | h | Measurement interval for on-line nutrient sensor |
| ODE scan time | $t_{scan}$ | constant | s | Frequency for integration of ODE model in between two measurements |
| Dead time | $t_{tot}$ | - | min | Parameter allowing to account for measuring process time and potential delay when measurements made in permeate line (travel from reactor to sensor) |
| Identity matrix | I | $\begin{bmatrix} 1 & 0 \\ 0 & 1 \end{bmatrix}$ | - | 2x2 matrix for Kalman filter (KF) |
| Observation Matrix | C | [1 0] | | Used to multiply only first element in KF |
| Error of on-line nutrient measurement | R | Constant | $(g/L)^2$ | Represents random noise of on-line measurement, i. e. variance. |
| System state | $X_{sys,t}$ | $\begin{bmatrix} c_{A,mess,t} \\ r_{A,t} \end{bmatrix}$ | - | Required to start control algorithm at $t = 0$ $t = n$: current interval $t = n \pm i$: previous or upcoming intervals |
| Var-Cov-Matrix of the state | Pt | $\begin{bmatrix} 0.01 & 0 \\ 0 & 0.01 \end{bmatrix}$ | - | The coefficient in (1,1) is the variance for glucose concentration and the coefficient in (2,2) is the variance for glucose uptake rate. (1,2) and (2,1) are covariances, that are not available in this case, so they are set to 0. This is updated with each cycle. The values indicated are the initial values used. |

Using the values listed in Table 1, an ODE model for the reaction rate in a continuous bioreactor is repeatedly integrated between the on-line glucose measurements. This allows to obtain calculated glucose concentrations consistently by digital simulation of the process. The model used in this example comprises equation (9) below:

$$\frac{dc_A}{dt} = \frac{F}{V}\left(c_{A,0} - c_A\right) - r_A \qquad (9)$$

which can also be described in matrix form with equation (10):

$$\dot{x}_{sys} = A \cdot x_{sys,t} + b \qquad (10)$$

where $A = \begin{bmatrix} -\frac{F}{V} & -1 \\ 0 & 0 \end{bmatrix}$ and $b = \begin{bmatrix} \frac{F}{V} \cdot C_{A,0} \\ 0 \end{bmatrix}$ with $\dot{x}_{sys}$ being the rate of change of the substrate concentration and substrate uptake rate in the bioreactor, $x_{sys,t}$ being the concentration of the substrate in the bioreactor at time t (t=n being the time with most recent data) and the substrate uptake rate at time t, F being the feed flow rate, $c_{A,0}$ being the substrate concentration in the feed flow rate, being the volume of the bioreactor and $r_A$ being the substrate uptake rate by the cells. The initial value of $r_A$ was set to a 'realistic' biological value for the applied cell line and media that was available from previous experiments. In this believed that the exact value of this does not have much impact within reasonable ranges, as this value is quickly updated with estimates from the Kalman Filter.

Indeed, with each new glucose measurement, new estimates of the system state $\hat{x}_{sys,t}$ are calculated using the Kalman filter (KF) described in equation (11) for the glucose concentration and glucose uptake rate:

$$\hat{x}_{sys,t} = x_{sys,t} + K_t \cdot (c_{A,mess,t} - C \cdot x_{sys,t}) \qquad (11)$$

Where $K_T$ is a Kalman gain defined as $K_T = P \cdot C^T \cdot (C \cdot P \cdot C^T + R)^{-1}$ (equation (11a)), $\hat{x}_{sys,t}$ is the vector of estimates of states of the system (i.e. the estimate of the glucose concentration in g/l and the estimate of the glucose uptake rate in g/l.h), C and R are parameters that are set by the user as explained in Table 1, and P is the covariance of the state which is updated at every iteration according to K and based on the previous value of P using: $P_t = (I - K \cdot C) \cdot P_{n-1}$ (equation (11b)). Thus, when a new on-line measurement is received, a new Kalman gain is obtained by comparing the new measurement with the system state obtained by integration of the ODE model (equation (9)) at the sampling time point. This is then used to calculate a new system state and to update the Variance-Covariance matrix P. Then, the model predictive controller takes over to calculate system estimates using equation (10) to calculate a control action minimizing the control objective J. Equation (11) estimates the system states based on weighing the measurement error by comparing the latest ODE predicted values (values obtained by integration of equation (10)) with the latest measurement value. Thus, the Kalman Gain K is used to weigh the deviation between on-line measurement and the state vector at the sampling time point to update / compute the new system state.

Using a continuous-time discrete KF allows to consider measurement dead times (potential dead volumes in tubings and measurement processing time) by calling the respective simulated glucose value derived from the ODE model. In other words, if a dead time of d seconds is expected then the value of $x_{sys,t}$ that is used may be a value that is associated with a time that is d seconds prior to the current measurement time, obtained by simulation of the ODE model from the preceding measurement time.

The glucose concentration estimate obtained as an output of the Kalman Filter is then used as input to a model predictive controller which solves the quadratic optimization problem of identifying values of the required change $\Delta u$ between two steps that minimize the cost function $J_k$ defined by equation (12) considering explicit constraints for step changes and width. The control variable u (feed flow rate) can then be adjusted accordingly.

$$J_k = \sum_{j=1}^{n} \left\| y_{k+j} - y_{ref,k+j} \right\|_Q^2 + \sum_{j=0}^{m-1} \left\| \Delta u_{k+j} \right\|_R^2 \qquad (12)$$

In equation (12), u is the manipulated variable (in this case feed flow rate in l/h), $\Delta u$ is the increment between two values of the manipulated variable, $y_{ref}$ is a reference trajectory for the controlled variable (in this case a set point for glucose concentration in g/l), $J_k$ is the cost function that is minimised at every iteration of the control method (i.e. $J_k$ is the value of the function at iteration k), y is the estimated value of the manipulated variable (in this case the glucose concentration in g/l, provided by the Kalman Filter), Q is a weighting matrix for the controlled variables penalties, R is a weighting matrix for the manipulated variables (controller outputs) penalties, k is the current time point, and k+j refers to one of a plurality of time points (prediction horizon) over which the function is optimised. In the present examples, the following values were used for Q and R: for the deviation of Glucose a weighing factor of 1 was used (Q) and for the deviation of the control action a weighing factor of 100 was used (R).

At the end of each iteration, a new feed flow rate is transferred from Matlab to MFCS/win 3.1 (i.e. in computing device 620) to the bioreactor control tower 650, e. g. Biostat® B-DCU from Sartorius, which is operated to control the pump rate of the perfusion media 680 accordingly.

*Experimental runs illustrating the functioning of the proof of concept model*

The concept described above was used to control the glucose concentration in Biostat® RM perfusion bioreactors in two separate experiments, that were designed to simulate the presence of disturbance (change in VCC setpoint - Run 1) and to test the performance of the method in the presence of a change in control behaviour (change in glucose setpoint - Run 2).

Run 1: the VCC setpoint was set at $20.10^6$ cells/ml up until day 7, and allowing growth to $40.10^6$ cells/ml thereafter. The glucose set point was set to a constant 5 g/l. As shown on **Figure 7A,** the perfusion was started at about $2.5 \cdot 10^6$ cells/ml at a perfusion rate of 1 vvd (total medium exchange rate, i.e. harvest / feed rate, **Figure 7D).** In this first phase glucose was measured daily off- and on-line. The APC algorithm was initialized after about 56 hours with an hourly on-line glucose measurement rate (phase 2). Shortly after, the target glucose concentration of 5 g/l was activated, such that the APC algorithm reduced the feed flow rate from 1 to 0.5 vvd (Figure 7D). As soon as the VCC was maintained at its first bleed setpoint of $20 \cdot 10^6$ cells/ml after 72 h, the glucose concentration was maintained at 5 g/l until day 10 **(Figure 7B).** During the period with the VCC setpoint of $20 \cdot 10^6$ cells/ml until 168 h, an average glucose uptake rate of 0.2 g (L h)-1 **(Figure 7C)** and slightly varying feed flow rates (Figure 7D) of mainly below 41.67 ml/h, i.e., 1 vvd were used. Changing the VCC setpoint to $40.10^6$ cells/ml after 168 h caused an increase in the glucose uptake rate (see Figure 7C) and to maintain the glucose setpoint, the feed flow rate was increased accordingly (see Figure 7D). This experiment demonstrates the ability of the controller to control the glucose setpoint (see the remarkable stability on Figure 7B) at different VCC setpoints and within growth phase inbetween changes of set points (see the targets steps on Figure 7A and the corresponding growth related changes in VCC).

Run 2: the VCC set point was set to a constant $40.10^6$ cells/ml, and the glucose setpoint was set to a setpoint of 5 g/l until day 8 and 4 g/l thereafter. Thus, the aim of this run was to demonstrate the ability of the described control approach to cope with a change in the glucose setpoint during the cultivation. Additionally, the feed flow rate was not started at 1 vvd as in run 1. Instead, the APC algorithm was used from the beginning to control the feed flow rate to maintain a glucose concentration of 5 g/l **(Figure 8B).** Therefore, initial flow values were at 10.42 ml/h, i.e., 0.25 vvd **(Figure 8D).** While the culture was growing to reach the target VCC of $40 \cdot 10^6$ cells/ml after 96 hours **(Figure 8A),** the feed flow rate automatically increased (Figure 8D) and the glucose uptake rate was calculated by the algorithm to increase as well **(Figure 8C).** The glucose setpoint of 5 g/L was maintained efficiently for 200 h (Figure 8B) with an average glucose uptake rate of 0.35 g/l.h (Figure 8C) and a feed flow rate of about 60 ml/h (Figure 8D). Then, the glucose setpoint was changed to 4 g/l, and the feed flow rate was decreased automatically by the APC algorithm (Figure 8D). This second setpoint was then maintained efficiently until the process end after 264 h (Figure 8B).

***Example 2***

*Introduction*

In this example, the control approach was used to maintain stable operation of a perfusion monoclonal (mAb) process throughout all phases of the process (i.e. from inoculation), using a controller that jointly controlled the VCD and volume.

*Methods*

The process was conducted in three stages:

1. Inoculation (days 0 - 3): No control actions taken, state observer updated in real time to monitor combined growth/death rate. Model predictions provided to operators.
2. Growth (days 3-7): Feed and harvest rates used to stabilize volume and achieve target cell specific perfusion rate.
3. Production (days 7-31): Feed, harvest, and bleed rates manipulated to achieve target setpoints for viable cell density, volume, and perfusion rate.

The experiment was conducted using CHO cells in a 2 liter Univessel™ perfusion vessel. Flow rates were regulated by adjusting pump speeds based on calibrated peristaltic pumps. Calibration was updated frequently based on changing vessel weights over time. Cell retention in the harvest line was achieved using an alternating tangential flow (ATF) filtration device. In this experiment, glucose was controlled manually by bolus additions made once per day as required to maintain suitable glucose levels.

In all stages except the inoculation stage, control actions were obtained by numerically solving the following minimization problem at a discrete control frequency of 2 hours:

$$\min_{F_f, F_h, F_b} p_x \left( \frac{x_v V}{V_{sp}} - x_{v,sp} \right)^2 + p_V \left( V - V_{sp} \right)^2 + p_p \left( F_f - F_{f,sp} \right)^2 + p_f \Delta F_f^2 + p_h \Delta F_h^2 + p_b \Delta F_b^2 + p_{VB} s_1 +$$
$$p_{VB} s_2 \quad (1f)$$

$$\frac{dx_v}{dt} = \left( \mu_{tot} + \frac{F_h - F_f}{V} \right) x_v \qquad (26)$$

$$\frac{dV}{dt} = F_f - F_h - F_b \qquad (27)$$

$$V - s_1 \leq Vmax \qquad (29)$$

$$V + s_2 \geq Vmin \qquad (30)$$

$$s_1, s_2 \geq 0 \qquad (31)$$

ODE (ordinary differential equation) constraints were enforced by discretizing the time domain using orthogonal collocation. At each sampling instant, the initial candidate feed, bleed and harvest rates were implemented on the real device.

The numerical optimization was calculated in Python on a Microsoft Azure™ virtual machine virtual machine and passed to the physical device via an OPC-UA protocol. User inputs both from control engineers and lab technicians were enabled by a web API and web-based graphical user interface.

The control tuning values used in this experiment (normalized to account for units of measurement so that the values represent relative importance in the objective function are shown in Table 2.

**Table 2.** Weights for control objective function (equation (1f)) used in the present example.

| | |
|---|---|
| $p_x$ | 3 |
| $p_v$ | 2 |
| $p_p$ | 2 |
| $p_f$ | 1 |
| $p_h$ | 3 |
| $p_b$ | 1 |
| $p_{VB}$ | $1 \times 10^8$ |

Note that this tuning heavily penalizes overflowing or emptying the vessel through $P_{VB}$. With the exception of this heavy penalty, note that the penalties on setpoint regulation have the same magnitude as those on input move suppression. As a consequence, the control objective heavily favours smooth, stable inputs to the process. This promotes an ideal environment for biological production compared to traditional control approaches where far more emphasis is placed on setpoint tracking than input move suppression.

**Figure 9A and 9B** demonstrate the achieved viable cell density and volume profiles over the 31 day run compared to the setpoint targets. Setpoint tracking performance was demonstrated by introducing a VCD setpoint change at day 24. **Figure 9C** shows the feed, harvest, and bleed rates proscribed by the controller. The high penalty on harvest and feed rate perturbation can be observed by the smoothness of the respective curves. **Figure 9D** plots the estimated growth/death rate, $\mu_{tot}$, calculated using a Luenberg observer.

*Equivalents and Scope*

All documents mentioned in this specification are incorporated herein by reference in their entirety. Unless context dictates otherwise, the descriptions and definitions of the features set out above are not limited to any particular aspect or embodiment of the invention and apply equally to all aspects and embodiments which are described. The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof. While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention, which is defined by the appended claims. Any section headings used herein are for organizational purposes only and are not to be construed as limiting the subject matter described. Any theoretical explanations provided herein are provided for the purposes of improving the understanding of a reader. The inventors do not wish to be bound by any of these theoretical explanations. "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein. As used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about" or "approximately", it will be understood that the particular value forms another embodiment. The terms "about" or "approximately" in relation to a numerical value is optional and means for example +/- 10%. Throughout this specification, including the claims, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps. Other aspects and embodiments of the invention provide the aspects and embodiments described above with the term "comprising" replaced by the term "consisting of" or "consisting essentially of', unless the context dictates otherwise.

**Claims**

1. A computer-implemented method for controlling a bioprocess comprising a cell culture in a bioreactor, wherein the bioprocess is operated as a perfusion bioprocess, the method comprising:

   controlling the value of one or more controlled variables selected from the number or density of viable cells in the bioreactor, the volume of culture in the bioreactor, and the concentration of one or more metabolites in the bioreactor using one or more control loops each configured to control a non-overlapping subset of the controlled variables by setting the value of one or more manipulated variables selected from the feed flow rate, the bleed flow rate and the permeate flow rate,
   wherein at least one of the control loops uses a controller that identifies a value of the one or more manipulated variables that optimises a control objective using a prediction of the value of the subset of controlled variables from a dynamic model of the bioprocess describing the rate of change of the one or more controlled variables, wherein the method comprises receiving a measurement of the value one or more controlled variables or a measurement from which the value of the one or more controlled variables can be derived, and automatically updating the value of one or more terms of the dynamic model of the bioprocess before predicting a state trajectory of the bioprocess, using the measurement or an estimate of the value derived from the measurement.

2. The method of claim 1, wherein the method is repeated every time a new measurement is received and/or wherein the estimate of the value is obtained using a state observer, and/or wherein the method comprises updating the value of one or more terms of the dynamic model of the bioprocess selected from a cell growth rate and a substrate uptake rate.

3. The method of claim 1 or claim 2, wherein controlling the value of the one or more controlled variables comprises controlling the value of each of:

   the number or density of viable cells in the bioreactor,
   the volume of culture in the bioreactor, and
   the concentration of one or more metabolites in the bioreactor; and/or

   wherein controlling the value of the one or more controlled variables comprises setting the value of each of: the feed flow rate, the bleed flow rate and the permeate flow rate.

4. The method of any preceding claim, wherein the control objective is an expression that penalises:

   differences between the prediction of the value of the subset of controlled variables and corresponding predetermined target values, and
   changes in the value of the one or more manipulated variables.

**5.** The method of any preceding claim, wherein the control objective is configured to penalise changes in the value of the one or more manipulated variables more strongly than differences between the prediction of the value of the subset of controlled variables and corresponding predetermined target values, and/or wherein the control objective is configured to prioritise stability of the manipulated variables, and/or wherein the control objective weights at least equally a term that penalises changes in the value of the one or more manipulated variables and a term that penalises differences between the prediction of the value of the subset of controlled variables and corresponding predetermined target values.

**6.** The method of any preceding claim, wherein the control objective comprises:

at least one term that penalises predicted values of a controlled variable that are below a predetermined first threshold, optionally wherein the term that penalises predicted values of a controlled variable that are below a predetermined first threshold penalises the difference between the predicted values of the controlled variable and the predetermined first threshold if said predicted values are below said predetermined first threshold; and/or at least one term that penalises predicted values of a controlled variable that are above a predetermined second threshold, optionally wherein the term that penalises predicted values of a controlled variable that are above a predetermined second threshold penalises the difference between the predicted values of the controlled variable and the predetermined second threshold if said predicted values are above said predetermined second threshold.

**7.** The method of any preceding claim, wherein the one or more controlled variables comprise the viable cell density, and the control objective uses the corresponding total number of cells instead of the viable cell density as control variable,
optionally wherein the control objective comprises a term that penalises differences between the prediction of the value of the total number of cells in the bioprocess and corresponding predetermined target values, instead or in addition to a term that penalises differences between the prediction of the value of the viable cell density in the bioprocess and corresponding predetermined target values.

**8.** The method of any preceding claim, wherein the method comprises using a state observer to provide an estimate of the value of one or more state variables of the bioprocess, the one or more state variables of the bioprocess comprising the one or more controlled variables, using one or more measurements of the state variables of the bioprocess and a dynamic model of the bioprocess describing the rate of change of said state variables,

optionally wherein the state observer provides an estimate of the value of one or more state variables of the bioprocess using a plurality of measurements for at least one of the state variables of the process, wherein the plurality of measurements include a first measurement and a second measurement, wherein the first measurement is acquired more frequently than the second measurement, and/or optionally wherein the state observer provides an estimate of the value of one or more state variables of the bioprocess using a measurement that is acquired with a time delay and wherein the state observer provides an estimate of the value of one or more state variables of the bioprocess that takes into account said time delay.

**9.** The method of any preceding claim, wherein the method comprises identifying one or more values of the one or more manipulated variables that minimises the control objective over a time period, and controlling the bioprocess to use one of the one or more values for each of the one or more manipulated variables, and/or wherein the controller that identifies a value of the one or more manipulated variables that optimises a control objective controls two or more controlled variable using a prediction of the value of the subset of controlled variables from a dynamic model of the bioprocess jointly describing the rate of change of the two or more controlled variables, and measurements of the value of the two or more controlled variables or estimates of the value of said variables obtained from said measurements.

**10.** The method of any preceding claim, wherein identifying a value of the one or more manipulated variables that optimises a control objective comprises using the dynamic model of the bioprocess to predict a state trajectory of the bioprocess with one or more candidate values of the one or more manipulated variables, optionally by finding values of the one or more state space variables that represent a solution of the dynamic model at one or more future time points, and using the prediction of the value of the subset of controlled variables in said state trajectory as variables of the control objective, optionally wherein the method comprises selecting and comparing candidate values using an optimisation algorithm.

**11.** The method of any preceding claim, wherein the state observer is a Kalman filter, a Luenberg observer or a moving horizon estimation, and/or

wherein the controlled variables comprise the viable cell density, volume and concentration of one or more metabolites, and/or
wherein the manipulated variables comprise the feed flow rate, the permeate flow rate, and the bleed flow rate, and/or
wherein the one or more control loops comprises:

a control loop that uses a controller that identifies a value of the one or more manipulated variables that optimises a control objective using a prediction of the value of the subset of controlled variables from a dynamic model of the bioprocess describing the rate of change of the one or more controlled variables; and one or more further control loops, optionally wherein the one or more further control loops do not use a dynamic model of the bioprocess and/or wherein the one or more further control loops use a controller selected from a proportional controller, a proportional-integral controller, and a proportional-integral-derivative controller.

12. The method of any preceding claim, wherein the manipulated variables include the permeate flow rate and the control objective comprises a term that penalises differences between candidate permeate flow rates and corresponding predetermined target values, a term that penalises candidate values of the permeate flow rate that are below a predetermined first threshold, and/or a term that penalises candidate values of the permeate flow rate that are above a predetermined second threshold, and/or
wherein the control objective comprises a term that penalises differences between candidate media exchange rates and corresponding predetermined target values, a term that penalises candidate values of the media exchange rate that are below a predetermined first threshold, and/or a term that penalises candidate values of the media exchange rate that are above a predetermined second threshold.

13. The method of any preceding claim, wherein the subset of controlled variables controlled by the control loop that uses a controller that identifies a value of the one or more manipulated variables that optimises a control objective using a prediction of the value of the subset of controlled variables from a dynamic model of the bioprocess describing the rate of change of the one or more controlled variables comprises:

(i) the concentration of one or more substrates, optionally wherein the one or more manipulated variables comprises the feed flow rate and/or wherein the one or more control loops further comprise a control loop, optionally using a P. PI or PID controller, that controls the volume as controlled variable using the permeate flow rate as manipulated variable, and a control loop, optionally using a P, PI or PID controller, that controls the viable cell density as controlled variable using the bleed flow rate as manipulated variable, and/or
(ii) the volume and viable cell density, optionally wherein the one or more manipulated variables comprises the permeate flow rate, the bleed flow rate and the feed flow rate.

14. The method of any preceding claim, further comprising one or more of:

obtaining a measurement for one or more of the controlled variables or a variable from which one or more of the controlled variables can be derived,
repeating the control method at one or more time points during operation of the bioprocess to determine corresponding values for the one or more manipulated variables,
repeating the control method at regular intervals during operation of the bioprocess to determine corresponding values for the one or more manipulated variables,
receiving a set of measurements for one or more of the controlled variables or variables from which one or more of the controlled variables can be derived and determining, using the one or more controller loops and the measurements, values of the manipulated variables, optionally wherein the step of determining is repeated every time a new set of measurements is received,
providing a set of values of the manipulated variables determined by the one or more control loops to a user, user interface, computer or actuator,
providing a signal to a control unit for control of one or more effectors to set the values of one or more manipulated variables according to a value determined by the one or more control loops,
receiving the value of one or more parameters of the controller, optionally from a computer device, data store, or user through a user interface, optionally wherein the parameters include parameters selected from: one or more predetermined targets for one or more controlled variables (such as e.g. set points for the one or more controlled variables; one or more of the predetermined targets may be variable during the course of the bioprocess, i.e. a different set point may be provided for each of a plurality of time points of the bioprocess), one or more parameters of the bioprocess (such as e.g. parameters related to the physical configuration of the bioprocess, physical constraints of the bioprocess, characteristics of the cell culture, temperature, etc.), one or more parameters of a dynamic model of the bioprocess (such as e.g. coefficients, initial values, etc.), one or more predetermined thresholds for one or more controlled variables (such as e.g. low/high thresholds below/above which a penalty is included in the objective function), a selection of one or more controlled variables, a selection of one or more manipulated variables, the value of one or more parameters of the control objective (such as e.g. the value of the weights applied to any of the terms of the control objective), and a selection of a state observer model (such as e.g. a Kalman Filter, a moving horizon estimator, a particular cost function for the moving horizon estimator, a dynamic model of the bioprocess to be used by the state observer, one or more parameters of the state observer such as the gain of a Kalman Filter, etc.), a time period for integration of the dynamic model of the bioprocess, and a frequency of iteration of the control method,
providing to a computing device, data store or user through a user interface, a prediction of the value of one or more states of the bioprocess determined by integration of the dynamic model of the bioprocess.

15. A system for controlling a bioprocess, the system including:

at least one processor; and
at least one non-transitory computer readable medium containing instructions that, when executed by the at least one processor, cause the at least one processor to perform the method of any of preceding claim;

optionally wherein the system further comprises, in operable connection with the processor, one or more of:

a user interface;
one or more biomass sensor(s);
one or more metabolite sensor(s);
one or more volume/level sensors;
a distributed control unit; and
one or more effector device(s), optionally wherein the one or more effector devices are operable to set the value of one or more manipulated variables.

Fig. 1

Fig. 2

Fig. 3A

Fig. 3B

Fig. 4A

Fig. 4B

Fig. 5

Fig. 6

Fig. 7A

Fig. 7B

Fig. 7C

Fig. 7D

Fig. 8A

Fig. 8B

Fig. 8C

Fig. 8D

Fig. 9A

Fig. 9B

Fig. 9C

Fig. 9D

Europäisches
Patentamt

European
Patent Office

Office européen
des brevets

**EUROPEAN SEARCH REPORT**

Application Number

EP 22 17 8451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/069180 A1 (SARTORIUS STEDIM DATA ANALYTICS AB [SE]) 7 April 2022 (2022-04-07) * page 17, line 13 – page 18, line 4; claims 1-4,11; figures 1a-1c * | 1-15 | INV. C12M1/34 C12M1/36 G16B40/00 |
| A | WO 2020/120232 A1 (GE HEALTHCARE BIO SCIENCES AB [SE]) 18 June 2020 (2020-06-18) * claim 1 * | 1-15 | |
| A | US 2020/066369 A1 (DOWNEY BRANDON JOHN [US] ET AL) 27 February 2020 (2020-02-27) * claim 1 * | 1-15 | |
| A | WO 2019/055796 A1 (BRISTOL-MYERS SQUIBB CO) 21 March 2019 (2019-03-21) * claim 1 * | 1-15 | |
| | | | TECHNICAL FIELDS SEARCHED (IPC) |
| | | | C12M G06F G16B G05B |

The present search report has been drawn up for all claims

| Place of search | Date of completion of the search | Examiner |
|---|---|---|
| Berlin | 16 November 2022 | Jones, Laura |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 8451

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2022

| Patent document cited in search report | | Publication date | Patent family member(s) | | Publication date |
|---|---|---|---|---|---|
| WO 2022069180 | A1 | 07-04-2022 | EP | 3979010 A1 | 06-04-2022 |
| | | | WO | 2022069180 A1 | 07-04-2022 |
| WO 2020120232 | A1 | 18-06-2020 | CN | 113168141 A | 23-07-2021 |
| | | | EP | 3894967 A1 | 20-10-2021 |
| | | | US | 2022010261 A1 | 13-01-2022 |
| | | | WO | 2020120232 A1 | 18-06-2020 |
| US 2020066369 | A1 | 27-02-2020 | EP | 3841193 A1 | 30-06-2021 |
| | | | US | 2020066369 A1 | 27-02-2020 |
| | | | WO | 2020041454 A1 | 27-02-2020 |
| WO 2019055796 | A1 | 21-03-2019 | CN | 111868223 A | 30-10-2020 |
| | | | EP | 3681989 A1 | 22-07-2020 |
| | | | JP | 2020533983 A | 26-11-2020 |
| | | | KR | 20200047702 A | 07-05-2020 |
| | | | US | 2020255785 A1 | 13-08-2020 |
| | | | WO | 2019055796 A1 | 21-03-2019 |

EPO FORM P0459

For more details about this annex : see Official Journal of the European Patent Office, No. 12/82

**REFERENCES CITED IN THE DESCRIPTION**

**Patent documents cited in the description**

- WO 2021165495 A
- US 20160152936 A
- WO 2014020327 A
- WO 2021165480 A

**Non-patent literature cited in the description**

- **ABBATE et al.** Experimental Validation of a Cascade Control Strategy for Continuously Perfused Animal Cell Cultures. *Processes,* 2020, vol. 8, 413